# EUROPEAN PATENT APPLICATION

(11) **EP 4 360 695 A1**
(43) Date of publication of application: **01.05.2024**
(21) Application number: 23203772.1
(22) Date of filing: 16.10.2023
(51) Int. Cl.: A61N 1/36, A61B 5/00

(54) **NOISE IDENTIFICATION AND REDUCTION FOR RECORDING OF SIGNALS WITH CARDIAC ACTIVITY ON SPINAL CORD STIMULATION (SCS) LEADS**

(30) Priority: 25.10.2022 US 202263419184 P; 27.09.2023 US 202318373841
(71) Applicant: Medtronic, Inc., Minneapolis, MN 55432 (US)
(72) Inventor: Straka, Malgorzata Maria, Blaine (US); Brinda, Annemarie K., Minneapolis (US); Hincapie, Juan G., Minneapolis (US); Litvak, Leonid M., Los Angeles (US); Mueller, Jerel Keith, Minneapolis (US); Nedrud, Joshua James, Minneapolis (US); Cleland, Andrew Jay, Minneapolis (US); Kharam, Aleksandra Pavlovna, Minneapolis (US); Dinsmoor, David A., Minneapolis (US)
(74) Representative: Zimmermann & Partner Patentanwälte mbB

(57) **Abstract**

A system is provided for identifying and reducing noise in a therapeutic procedure. For example, the system may be configured to measure one or more signals with cardiac activity. The system may be configured to then identify one or more sources of noise that are distorting the one or more signals with cardiac activity and may reduce the one or more sources of noise from the one or more signals with cardiac activity. Subsequently, the system may be configured to determine one or more aggregate cardiac-derived metrics and/or save processed cardiac data based on the one or more signals with cardiac activity with the one or more sources of noise reduced. Additionally, the system may be configured to determine one or more parameters for applying an electrical signal to an anatomical element of a patient based on the one or more aggregate cardiac-derived metrics and/or saved processed cardiac data.

## Description

### CROSS-REFERENCE TO RELATED APPLICATIONS

This application claims the benefit of U.S. Provisional Application No. 63/419,184, filed on October 25, 2022, which application is incorporated herein by reference in its entirety.

### BACKGROUND

The present disclosure is generally directed to therapeutic neuromodulation, and relates more particularly to detecting signals with cardiac activity for supporting the therapeutic neuromodulation.

Neuromodulation therapy may be carried out by sending an electrical signal generated by a device (e.g., a pulse generator) to a stimulation target (e.g., nerves, non-neuronal cells, etc.), which may provide a desired electrophysiologic, biochemical, or genetic response in the stimulation target. Neuromodulation therapy systems may be used to deliver electrical stimulation for providing chronic pain treatment to a patient. In some neuromodulation therapies (e.g., closed-loop neuromodulation therapies), one or more signals resulting from the neuromodulation may be recorded and the therapy may be adjusted based on the recorded signals. Additionally or alternatively, the recorded signals may be used for monitoring and/or indicating conditions of the patient.

### BRIEF SUMMARY

Example aspects of the present disclosure include:
A system for identifying and reducing noise in a therapeutic procedure, comprising: a pulse generator configured to generate a therapeutic electrical signal; one or more leads in communication with the pulse generator and configured to transmit the therapeutic electrical signal to a plurality of electrodes; the plurality of electrodes in communication with the one or more leads, the plurality of electrodes configured to apply the therapeutic electrical signal to an anatomical element of a patient and configured to measure a physiological response; a processor; and a memory storing data for processing by the processor. In some embodiments, the data, when processed, may cause the processor to: measure, via one or more of the plurality of electrodes, one or more signals with cardiac activity of the patient; identify one or more sources of noise that are distorting the one or more signals with cardiac activity; reduce the one or more sources of noise from the one or more signals with cardiac activity; determine one or more aggregate cardiac-derived metrics and/or save processed cardiac data based at least in part on the one or more signals with cardiac activity with the one or more sources of noise reduced; and determine one or more parameters for applying the therapeutic electrical signal to the anatomical element based at least in part on the one or more aggregate cardiac-derived metrics and/or saved processed cardiac data.

Any of the aspects herein, wherein the memory stores further data for processing by the processor that, when processed, causes the processor to: process one or more portions of the one or more signals with cardiac activity, the one or more portions corresponding to time points when the one or more sources of noise are expected to occur after the therapeutic electrical signal is applied to the anatomical element.

Any of the aspects herein, wherein the one or more portions of the one or more signals with cardiac activity are blanked immediately before, during, and/or after individual pulses of the therapeutic electrical signal are applied to the anatomical element.

Any of the aspects herein, wherein the one or more portions of the one or more signals with cardiac activity are processed based at least in part on a template subtraction method, spectral filtering, wavelet filtering, or a combination thereof.

Any of the aspects herein, wherein the memory stores further data for processing by the processor that, when processed, causes the processor to: generate one or more growth curves based at least in part on applying the therapeutic electrical signal to the anatomical element; determine a threshold based at least in part on the one or more growth curves, wherein the one or more aggregate cardiac-derived metrics are collected when evoked signals are below the determined threshold; and perform signal processing for stimuli that evoke signals larger than the threshold to measure the one or more signals with cardiac activity, wherein the one or more sources of noise are reduced based at least in part on performing the signal processing for stimuli that evoke signals larger than the threshold.

Any of the aspects herein, wherein the stimuli that evoke signals larger than the threshold comprise stimulation amplitudes for added processing.

Any of the aspects herein, wherein the data stored in the memory that, when processed causes the processor to reduce the one or more sources of noise from the one or more signals with cardiac activity further causes the system to: cycle one or more parameters used for applying the therapeutic electrical signal to the anatomical element and/or one or more parameters for measuring the one or more signals with cardiac activity between 'on' phases and 'off' phases, wherein the one or more signals with cardiac activity are measured during the 'off' phases.

Any of the aspects herein, wherein the 'on' phases comprise applying the therapeutic electrical signal to the anatomical element using a combination of high frequency and/or low frequency stimulations and/or high and/or low amplitudes, and the 'off' phases comprise applying the therapeutic electrical signal to the anatomical element using low frequency stimulations, lower ratios of cycling, low amplitudes, or a combination thereof.

Any of the aspects herein, wherein the data stored in the memory that, when processed causes the processor to reduce the one or more sources of noise from the one or more signals with cardiac activity further causes the system to: filter the one or more signals with cardiac activity to reduce the one or more sources of noise.

Any of the aspects herein, wherein the data stored in the memory that, when processed causes the processor to reduce the one or more sources of noise from the one or more signals with cardiac activity further causes the system to: identify time points when the one or more signals with cardiac activity have been corrupted by the one or more sources of noise; and remove portions of the one or more signals with cardiac activity corresponding to the identified time points to reduce the one or more sources of noise from the one or more signals with cardiac activity.

Any of the aspects herein, wherein the time points are identified based at least in part on time intervals when a given R-R interval is non-physiological and/or differs from R-R intervals detected for preceding heartbeats by a defined threshold, when a presence of increased spectral power of greater than a frequency threshold is identified, when changes in a magnitude threshold for the one or more signals with cardiac activity are identified, when movement is detected for the patient, or a combination thereof.

Any of the aspects herein, wherein the memory stores further data for processing by the processor that, when processed, causes the processor to: output, via a user interface, the one or more aggregate cardiac-derived metrics and/or the saved processed cardiac data, wherein the one or more parameters for applying the therapeutic electrical signal to the anatomical element are determined based at least in part on outputting the one or more aggregate cardiac-derived metrics and/or the saved processed cardiac data.

Any of the aspects herein, wherein the one or more signals with cardiac activity of the patient are measured using a continuously running noise filter, wherein the noise filter is adjusted when the one or more sources of noise meet or exceed a predetermined threshold, wherein the adjusted noise filter uses more power than the non-adjusted noise filter.

Any of the aspects herein, wherein the one or more signals with cardiac activity are measured when the patient is stationary or in a specific position.

Any of the aspects herein, wherein the one or more sources of noise are caused by the patient walking or moving, a stimulation artifact, environmental noise, evoked compound action potential activity, evoked compound muscle action potential activity, or a combination thereof.

A system for identifying and reducing noise in a therapeutic procedure, comprising: a processor; and a memory storing data for processing by the processor, the data, when processed, causes the processor to: measure, via one or more of a plurality of electrodes, one or more signals with cardiac activity of a patient; identify one or more sources of noise that are distorting the one or more signals with cardiac activity; reduce the one or more sources of noise from the one or more signals with cardiac activity; determine one or more aggregate cardiac-derived metrics and/or save processed cardiac data based at least in part on the one or more signals with cardiac activity with the one or more sources of noise reduced; and determine one or more parameters for applying a therapeutic electrical signal to an anatomical element based at least in part on the one or more aggregate cardiac-derived metrics and/or saved processed cardiac data.

Any of the aspects herein, wherein the memory stores further data for processing by the processor that, when processed, causes the processor to: process one or more portions of the one or more signals with cardiac activity, the one or more portions corresponding to time points when the one or more sources of noise are expected to occur after the therapeutic electrical signal is applied to the anatomical element.

Any of the aspects herein, wherein the one or more portions of the one or more signals with cardiac activity are blanked immediately before, during, and/or after individual pulses of the therapeutic electrical signal are applied to the anatomical element.

Any of the aspects herein, wherein the memory stores further data for processing by the processor that, when processed, causes the processor to: generate one or more growth curves based at least in part on applying the therapeutic electrical signal to the anatomical element; determine a threshold based at least in part on the one or more growth curves, wherein the one or more aggregate cardiac-derived metrics are collected when evoked signals are below the determined threshold; and perform signal processing for stimuli that evoke signals larger than the threshold to measure the one or more signals with cardiac activity, wherein the one or more sources of noise are reduced based at least in part on performing the signal processing for stimuli that evoke signals larger than the threshold.

Any of the aspects herein, wherein the data stored in the memory that, when processed causes the processor to reduce the one or more sources of noise from the one or more signals with cardiac activity further causes the system to: cycle one or more parameters used for applying the therapeutic electrical signal to the anatomical element between 'on' phases and 'off' phases, wherein the one or more signals with cardiac activity are measured during the 'off' phases.

A system for identifying and reducing noise in a therapeutic procedure, comprising: a pulse generator configured to generate a therapeutic electrical signal; one or more leads in communication with the pulse generator and configured to transmit the electrical signal to a plurality of electrodes; and the plurality of electrodes in communication with the one or more leads, the plurality of electrodes configured to apply the electrical signal to an anatomical element of the patient and configured to measure a physiological response, wherein the therapeutic electrical signal is applied to the anatomical element according to one or more aggregate cardiac metrics and/or saved processed cardiac data derived from one or more parameters determined from one or more signals with cardiac activity measured via one or more of the plurality of electrodes, the one or more signals with cardiac activity having one or more sources of noise reduced from the one or more signals with cardiac activity.

Any of the aspects herein, wherein the one or more sources of noise are caused by the patient walking or moving, a stimulation artifact, environmental noise, evoked compound action potential activity, evoked compound muscle action potential activity, or a combination thereof.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a set of example signal recordings according to at least one embodiment of the present disclosure;
Fig. 3 is an example recorded signal with noise present according to at least one embodiment of the present disclosure;
Fig. 4 is a set of example signal recordings including electrical stimulation artifacts according to at least one embodiment of the present disclosure;
Fig. 5 is a set of example signal recordings for a patient while the patient is walking according to at least one embodiment of the present disclosure;
Fig. 6 is an example recorded signal with noise present according to at least one embodiment of the present disclosure;
Fig. 7 is an example spectrogram of filtered signals according to at least one embodiment of the present disclosure;
Fig. 8 is an example recorded signal with noise present according to at least one embodiment of the present disclosure;
Fig. 9 is a diagram of a system according to at least one embodiment of the present disclosure;
Fig. 10 is a flowchart of a method according to at least one embodiment of the present disclosure;
Fig. 11 is a flowchart of a method according to at least one embodiment of the present disclosure; and
Fig. 12 is a flowchart of a method according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., random-access memory (RAM), read-only memory (ROM), electrically erasable programmable ROM (EEPROM), flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Cortex Mx; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to a device, operator, or user of the system, and distal being further from the device, operator, or user of the system.

For some closed-loop neuromodulation therapies, a therapeutic electrical signal generated by a pulse generator may be sent to a stimulation target (e.g., nerves, non-neuronal cells, etc.). A biopotential (e.g., recorded signal) elicited with the therapeutic electrical signal may be recorded. The elicited biopotential may provide information by which to adjust the therapeutic electrical signal. Other types of closed-loop neuromodulation therapies may use and sense other types of signals to determine adjustments for the therapeutic electrical signal, such as outputs of other sensors implanted in or placed on a patient (e.g., posture sensor, accelerometer, etc.).

In some examples, spinal cord stimulation (SCS) (e.g., a form of neuromodulation that includes applying a therapeutic electrical signal or stimulation signal to nerves of the spinal cord or nerves near the spinal cord to elicit a desired electrophysiologic, biochemical, or genetic response) may be practiced in a closed-loop manner. When SCS is performed in a closed-loop manner, contacts (e.g., leads, electrodes, etc.) may be placed near a stimulation target (e.g., patient's spinal cord or a proximate structure (such as the dorsal root ganglion) or one or more targets), where the contacts are configured to apply a therapeutic electrical signal to the stimulation target to obtain a desirable electrophysiologic, biochemical, or genetic state (e.g., that leads to pain relief). For example, the therapeutic electrical signal may be configured to change how the patient's body interprets a pain signal based on causing a desired electrophysiologic, biochemical, or genetic response when applied to the stimulation target. During SCS, the patient may move, and thus the stimulation target may also move further away from or closer to the contacts and thus affect the extent of stimulation energy coupled to the stimulation target.

To mitigate such undesirable effects, some contacts and/or an additional device of an SCS system may be configured to record one or more signals (e.g., biopotentials, outputs of sensors, etc.) that are generated based on applying the therapeutic electrical signal, and the recorded signal(s) may be used to determine and/or adjust parameters of the therapeutic electrical signal. For example, some contacts may be used to stimulate the nerve and other contacts may be used to record an evoked response, such as the Evoked Compound Action Potential (ECAP) or the Evoked Compound Muscle Action Potential (ECMAP), resulting from the stimulation. The ECAP recording can be used to adjust the stimulation in real-time to compensate for movement of the spinal cord to and from the contacts. In other embodiments it may be desirable to record the ECAP whether with movement or without movement as the ECAP may provide information about a pathophysiology of a patient. In such embodiments, the ECAP may also be used to modulate therapy provided based on the ECAP signal. In other words, the ECAP may be used to modulate therapy whether or not there is movement of the patient, contacts, anatomical elements, stimulation target, etc. In other closed-loop neuromodulation therapies, other signals may be recorded. For example, in deep brain stimulation, local field potentials (LFPs) may be recorded in the brain. In various types of therapies provided, the desired signal to be recorded (e.g., ECAPs, LFPs, etc.) may be recorded at a distance from the stimulation, near the stimulation, or from multiple places. However, the recording may include stimulation induced electrical artifacts, which may mask, obscure, or otherwise corrupt at least a portion of the recording and thus, may interfere with the cardiac measures and/or the adjusted therapy provided.

As described previously, SCS stimulation may utilize a closed-loop neuromodulation therapy by using ECAPs to refine the delivery of therapy. However, there are many other biomarkers related to chronic pain, including physiological signals like Heart Rate (HR) and Heart Rate Variability (HRV). Specifically, the chronic pain state has been correlated with an increase in baseline HR and a decrease in HRV. Calculated HRV metrics may include (but are not limited to) time series metrics, such as standard deviation N-N (SDNN) intervals of normal heart beats and the root mean squared of successive differences (RMSSD) between normal heart beats, frequency domain metrics, such as high-frequency and low frequency HRV, and time-frequency metrics. These HRV metrics provide different insights into autonomic nervous system function. For example, RMSSD and high frequency HRV are associated with parasympathetic activity. In some embodiments, SCS therapy has been shown to bring these biomarkers closer to a normal range, corresponding to increased pain relief.

Physiological signals, including cardiac electrogram signals can be recorded on SCS leads and be used to determine relevant metrics such as HR, HRV, respiration, etc. Previous techniques have been developed for recording signals that indicate cardiac activity from or on SCS leads. Additionally, these techniques include general discussion of filtering and removing ECAP signals from recorded signals, but no specific potential methods for either determining when to remove or methods for remove the ECAP signals have been developed or provided. Additionally, the previously developed techniques do not include determining when and/or providing methods for removing other types of signals from the recorded signals, such as ECMAPs. The previously developed techniques also do not consider how to identify, determine when to remove, or provide methods for removing movement artifacts when analyzing the recorded signals. Additionally, the previously developed techniques do not discuss using growth curves to determine thresholds of when to apply ECAP removal techniques. While the previously developed techniques consider leveraging cycling off times to record the signals, additional details (e.g., such as cycling high frequencies for therapies like differential target multiplexing (DTM^{™})) are not provided.

The physiological signals can be detected in various positions. Accordingly, the physiological signals may be processed to better identify key features for the patient, which may include R-peaks (e.g., maximum amplitude of an R wave, where the R wave represents a component of electrical activity of the heart as it passes through an anatomical element of the patient).

However, the physiological signals (e.g., cardiac electrogram signal(s)) may be easily corrupted by a variety of confounding signals, which include electrical aggressors such as muscle noise or evoked activity from stimulation (e.g., ECAPs, Evoked Compound Muscle Action Potential (ECMAPs), etc.). To accurately calculate cardiac-derived measures (e.g., which may include HR, HRV, RMSSD between normal heartbeats, frequency-domain information of the heart, QRS width, QT duration, PR interval, respiration, etc.), non-cardiac signals must be identified, reduced, and removed.

There are three main sources of unwanted noise/signals that may obstruct cardiac signals. The first source may include artifacts due to electrical stimulation. The second source may include physiological signal responses elicited by stimulation, which may include ECAPs, ECMAPs, other evoked action potentials, etc. The physiological signal responses are time-synchronized to a stimulation pulse and often have characteristic relationships to the stimulation amplitude. The third source may include complex signals that can be describe as 'movement artifacts,' given that the signals are often present while the subject is moving; far-field detection of EMG is a common source of this noise type. These sources of noise may be identified and removed through one or a combination of methods including, but not limited to, blanking, template subtraction, common-mode signal rejection, filtering (e.g., spectral filtering, wavelet filtering, etc.), principal component analysis, and/or regression methods.

As described herein, an algorithm is provided that reduces and identifies unwanted noise and/or signals that obstruct the detection of cardiac signals recorded on SCS leads in order to accurately and reproducibly analyze cardiac-derived measures for patient monitoring and/or for supporting a closed-loop neuromodulation therapy. The algorithm describes methods that may be used independently or in conjunction for removing evoked physiological signals and/or movement artifact from obtained cardiac metrics.

In one embodiment, for stimulation evoked signals, one method may include processing or blanking of the samples at which the stimulation evoked signals are or may be present. For example, ECAPs on the spinal cord are usually present at latencies of 0-3 milliseconds (ms) after the therapeutic electrical signal (e.g., stimulation) is applied to the spinal cord (e.g., or to nearby nerves), so the corresponding time points according to the latencies may be blanked or processed using methods including template subtraction (i.e., where the contribution of an artifact is approximated as a template before being subtracted from the signal), spectral filtering, wavelet filtering, or a combination thereof. In some examples, the stimulation evoked signals (e.g., recorded signals that include cardiac activity) may be processed or blanked at the corresponding time points based at least in part on removing, attenuating, or replacing portions of the stimulation evoked signals at the corresponding time points with background activity with either hardware, firmware, and/or software methods. In some embodiments, the processing or blanking may be applied before, during, and/or after each pulse.

Additionally or alternatively, growth curves of evoked activity may be collected to determine ranges that do or do not need processing for noise removal. For example, growth curves could be used to determine ECAP thresholds, and the signal processing could be applied exclusively after stimulation amplitude exceeds a certain percentage of the ECAP threshold (e.g., 10% above the ECAP threshold). The signal processing may also be used for ECMAPs or other evoked signals at relevant latencies after stimulation. Another option for reducing stimulation artifacts is to cycle stimulation and/or recording parameters between ON phases and OFF phases. In one embodiment, such as DTM^{™} SCS, high frequency stimulation may be cycled while low frequency stimulation is continuous and/or cycled at lower ratios. In another embodiment, all stimuli are cycled. In the OFF phase of cycling, physiological signals may be collected so as to avoid contamination of stimulation artifacts. Additionally or alternatively, the therapy may be configured or timed based on cardiac pulses of the patient. For example, therapy pulses may be delivered during a cardiac refractory period and/or in between cardiac pulses to reduce noise from the one or more measured and/or recorded signals with cardiac activity. In some embodiments, noise may be reduced from recorded signals (e.g., signals with cardiac activity as described herein) based on selecting electrodes configured for recording the signals such that noise is minimized.

Additionally or alternatively, the algorithm provided herein may reduce or remove non-cardiac signals that may include movement artifacts with or without the presence of evoked response noise from measured cardiac signals using signal processing. In some embodiments, filtering techniques may be used to remove or reduce the non-cardiac signals from the measured cardiac signals. In other embodiments, the presence of non-cardiac signals may impact the fidelity of the cardiac signal, especially since the spectral power of movement artifacts overlaps with cardiac signals. In such embodiments, the corrupted signal must be identified and removed. There are a number of ways to identify time points when the measured cardiac signals (e.g., ECG signal(s)) have been corrupted, which include any one and/or combination of:
- When an R-R interval is non-physiological and/or differ by greater than a defined threshold (e.g., more than 60% different) than R-R interval(s) detected by preceding heartbeats;
- The presence of increased spectral power at frequencies higher than a frequency threshold value (e.g., approximately 50 Hertz (Hz) or greater);
- A magnitude threshold may be used to track the magnitude of the raw or filtered signal with cardiac activity, and changes in the magnitude threshold may indicate areas with unacceptable noise;
- The use of an accelerometer to detect movement and remove sections of data with movement and/or only analyze cardiac signals when the patient is still or in a specific position; and/or
- Other spectral or temporal algorithms that may identify the presence of noise using techniques such as wavelet analysis, principal component analysis, etc.

Additionally or alternatively, the algorithm provided herein may also use 'common-mode' signals, or signals that are common across a plurality of electrodes, to identify signals with cardiac activity and/or remove noise.

Embodiments of the present disclosure beneficially enable identification and reduction of noise when measuring cardiac metrics and movements of a patient to inform an implantable neuromodulation or neurostimulation system (e.g., SCS system). Embodiments of the present disclosure also beneficially enable a user-friendly neuromodulation system that performs closed-loop adjustments without the addition of extra devices (e.g., wearables) and/or inputs from the patient. Embodiments of the present disclosure further beneficially enable an improved SCS therapy based on obtaining accurate cardiac measurements of a patient with noise reduced to enable closed-loop adjustments of the SCS therapy.

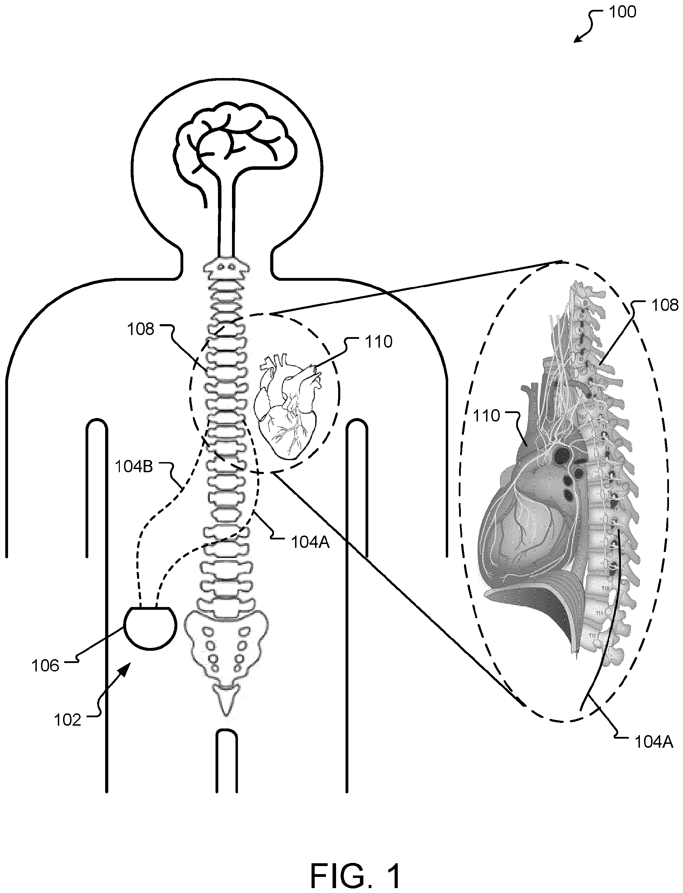

Turning to Fig. 1, a diagram of aspects of a system 100 according to at least one embodiment of the present disclosure is shown. The system 100 may be used to provide electric signals for a patient and/or carry out one or more other aspects of one or more of the methods disclosed herein. For example, the system 100 may include at least a device 102 that is capable of providing a stimulation applied to the spinal cord 108 of the patient and/or to one or more nerve endings for a patient (e.g., for SCS therapy). In some examples, the device 102 may be referred to as an implantable pulse generator 106. More specifically, the implantable pulse generator 106 may be configured to generate a current or therapeutic electrical signal, such as a signal capable of stimulating a response in the spinal cord 108 or from one or more nerves. In some embodiments, as described herein, the device 102 may be implanted within the patient.

Additionally, the system 100 may include one or more leads 104 (e.g., electrical leads) that provide a connection between the device 102 and the spinal cord or nerves of the patient for enabling, for example, stimulation. In some embodiments, the leads 104 may be implanted wholly or partially within the patient.

In some embodiments, the one or more leads 104 may include a first lead 104A disposed on or connected to a first side of the spinal cord 108 of the patient and a second lead 104B disposed on or connected to a second side of the spinal cord 108 of the patient. For example, the first lead 104A may be connected to the righthand side of the spinal cord 108, while the second lead 104B may be connected to the lefthand side of the spinal cord 108. However, the position and/or orientation of each lead relative to the spinal cord 108 may vary depending on, for example, the type of treatment, the type of lead, combinations thereof, and the like. In another example, the first lead 104A and the second lead 104B may overlap one another, and may be placed proximate one another on the dorsal side of the spinal cord 108 close to a midline of the spinal cord 108. In some examples, the first lead 104A and the second lead 104B may both be placed on the midline of the spinal cord 108, where one of the leads 104 is cranial (e.g., anterior or nearer the head of the patient) and the other of the leads 104 is caudal (e.g., posterior or nearer the tail of the patient). Additionally or alternatively, the first lead 104A and the second lead 104B may both be placed on one side of the midline of the spinal cord 108.

Additionally, the one or more leads 104 may be connected, placed, or otherwise implanted near or on the spinal cord 108 within the patient, such that at least one of the one or more leads 104 are located near the heart 110 of the patient. For example, as shown in the inset or zoomed-in portion of the example of Fig. 1 which depicts a side view of the encircled area within the patient, the first lead 104A may be placed within the spinal canal behind the heart 110 (e.g., dorsally within the spinal canal, such as behind a foramen of the spine near the top of a vertebra of the thoracic vertebrae column of the spinal cord 108, or anteriorly within the spinal canal). Additionally or alternatively, as described previously, the exact placement of the one or more leads 104 may vary depending on, for example, the type of treatment, the type of lead, the patient, combinations thereof, and the like. While not specifically shown in the example of Fig. 1, the one or more leads 104 may also exit the spinal cord 108 at a lumbar vertebra lower down the spinal cord 108 (e.g., the L2 vertebra, but the exact location may vary). As described herein, the one or more leads 104 being placed proximate to the heart 110 may enable the system 100 to more effectively capture signals that include cardiac activity before, during, and/or after providing a neuromodulation therapy (e.g., SCS therapy).

In other embodiments, the one or more leads 104 may include at least the first lead 104A and the second lead 104B connected to other nerves of the patient (e.g., the vagus nerve, different trunks of the vagus nerve, etc.). For example, the first lead 104A may be connected to a first nerve (e.g., first vagal trunk of the patient, such as the anterior sub diaphragmatic vagal trunk at the hepatic branching point of the vagus nerve) and the second lead 104B may be connected to a second nerve (e.g., second vagal trunk of the patient, such as the posterior sub diaphragmatic vagal trunk at the celiac branching point of the vagus nerve). The first lead 104A and/or the second lead 104B may be configured to provide an electrical stimulation signal from the device 102 to the respective first and/or second nerve. The connection of the leads 104 to the respective nerve (or other nerves) of the patient may permit the device 102 to measure and/or stimulate one or more evoked potentials (e.g., ECAPs) in the patient based on the provided electrical stimulation from the implantable pulse generator 106.

In some examples, the leads 104 may provide the therapeutic electrical signals to the respective nerves via electrodes or electrode devices that are connected to the nerves (e.g., sutured in place, wrapped around the nerves, etc.). In some examples, the leads 104 may be referenced as cuff electrodes or may otherwise include the cuff electrodes (e.g., at an end of the leads 104 not connected or plugged into the device 102). For example, electrodes, electrode devices, cuff electrodes, paddle electrodes, or a different type of electrode may be disposed at a distal end of each of the leads 104.

In other examples, the leads 104 may be or comprise linear SCS leads capable of delivering one or more stimulation signals (e.g., generated by the device 102) to the spinal cord 108. The leads 104 may comprise a plurality of electrodes disposed along the length of the lead, such that the leads 104 contact the spinal cord 108 at multiple points along a length of the spinal cord 108. A first set of the electrodes on each lead may pass an electrical signal into the spinal cord 108, while a second set of the electrodes on each lead may sense one or more signals generated in response by the spinal cord 108 (e.g., recorded signals). In one embodiment, the electrodes may be able to sense, measure, or otherwise collected data related to ECAPs (e.g., ECAP waveforms). Additionally or alternatively, the electrodes may be able to sense, measure, or otherwise collected data related to cardiac metrics for the patient (e.g., HR, HRV, respiration, or other ECG measurements). In some examples, the device 102 may be used as a contact and/or may include additional contacts for sensing, measuring, or otherwise collecting data related to cardiac metrics for the patient. A plurality of these configurations can be used to record different heart vectors of cardiac activity towards deriving various cardiac metrics.

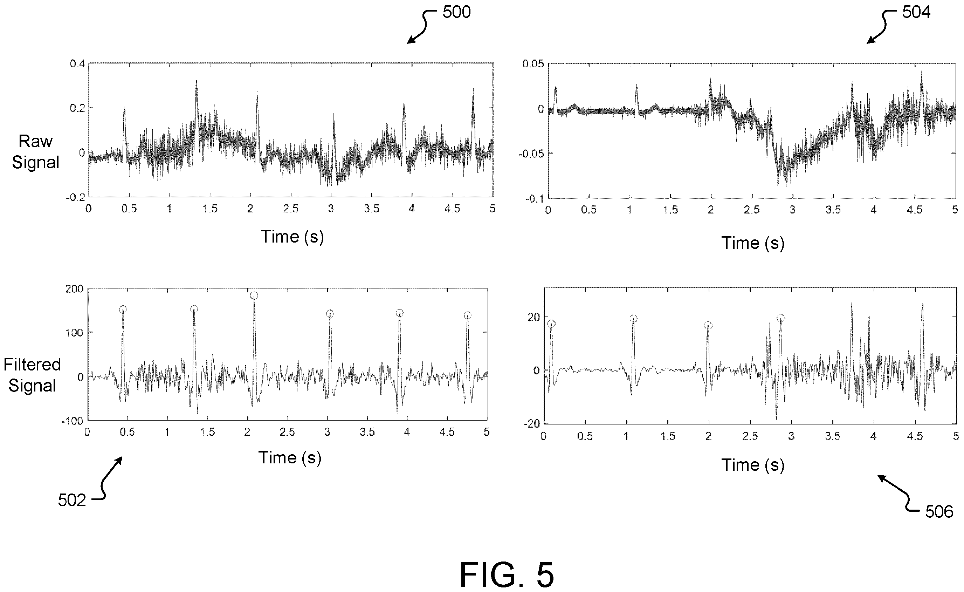

Additionally, while not shown, the system 100 may include one or more processors (e.g., one or more DSPs, general purpose microprocessors, graphics processing units, ASICs, FPGAs, or other equivalent integrated or discrete logic circuitry) shown and described in Fig. 5 that are programmed to carry out one or more aspects of the present disclosure. In some examples, the one or more processors may include a memory or may be otherwise configured to perform the aspects of the present disclosure. For example, the one or more processors may provide instructions to the device 102, the leads 104, the electrodes, or other components of the system 100 not explicitly shown or described with reference to Fig. 1 for applying a stimulation, performing measurements (e.g., cardiac metrics, ECAPs, etc.), and analyzing the same, as described herein. In some examples, the one or more processors may be part of the device 102 or part of a control unit for the system 100 (e.g., where the control unit is in communication with the device 102 and/or other components of the system 100).

As described herein, the system 100 (e.g., an SCS system) may implement an algorithm that reduces and identifies unwanted noise and/or signals that obstruct the detection of cardiac signals recorded on the leads 104 and/or electrodes in order accurately and reproducibly analyze cardiac-derived measures. The cardiac-derived measures may then be used to determine and/or adjust one or more parameters for applying the therapeutic electrical signal to the spinal cord 108 (e.g., or nearby nerves) to optimally provide pain treatment for the patient (e.g., as part of a closed-loop neuromodulation therapy). For example, chronic pain state has been correlated with an increase in baseline HR and decrease in HRV (e.g., cardiac electrogram signal(s)), and the SCS therapy provided by the system 100 can bring these biomarkers closer to normal range to cause or correspond with increased pain treatment. Accordingly, accurate cardiac measurements (e.g., with the unwanted noise and/or signals removed or reduced) are needed to enable an optimal closed-loop system. Additionally or alternatively, the cardiac-derived measures may be provided (e.g., to a physician or other medical practitioner) for patient monitoring.

The device 102 may be programmed to measure and record movements of the patient (e.g., for the purpose of life, sleep, and activity tracking). For example, the device 102 may comprise an accelerometer and/or other components that are designed to track and record movements of the patient (e.g., whether the patient is moving, not moving, laying down, standing up, running, walking, etc.). Additionally, the leads 104 and/or electrodes disposed at the distal end of the leads 104 may be programmed to measure a physiological response of the patient. In some examples, the physiological response may comprise an evoked response (e.g., ECAP measurement) based on applying the therapeutic electrical signal (e.g., stimulation signal) generated by the device 102 to the spinal cord 108 (e.g., and/or to nearby nerves as described previously). Additionally or alternatively, as described herein, the physiological response may comprise cardiac signals (e.g., HR, HRV, respiration, other cardiac electrogram-related measurements, etc.) of the patient before and after the therapeutic electrical signal is applied. In some examples, the device 102 may be programmed to measure and record the cardiac signals (e.g., via an electrode vector and/or electrodes placed on an outer surface of the device 102 and/or within the device 102) in addition or alternative to the leads 104 and/or electrodes. Additionally or alternatively, an additional device (e.g., implanted within the patient, an external device, etc.) may be configured or programmed to record cardiac activity of the patient.

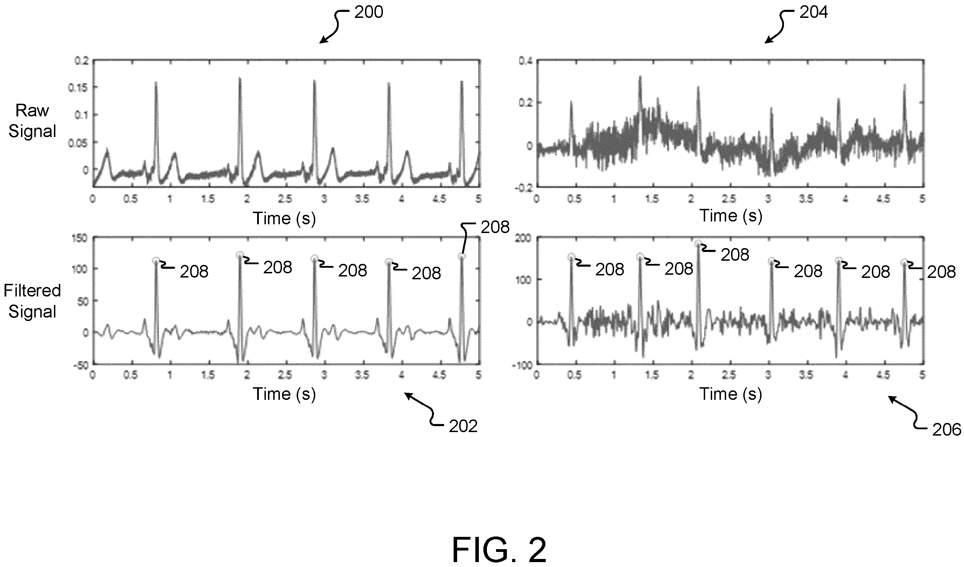

Fig. 2 is a set of example signal recordings with cardiac activity according to at least one embodiment of the present disclosure. In some examples, the set of example signal recordings may be acquired by one or more aspects of Fig. 1. For example, the set of example cardiac signal recordings may represent physiological signals (e.g., cardiac electrogram signals) that can be recorded by components of the system 100 (e.g., SCS leads, such as the leads 104 and/or the electrodes). The physiological signals can be detected in various positions of the patient. Additionally, the physiological signals may be processed to better identify key features, which may include R-peaks.

A first signal recording 200 may represent a raw signal with cardiac activity recorded from SCS leads in ideal conditions when the patient is seated with no stimulation being applied. A first filtered signal recording 202 may represent a filtered version of the first signal recording 200 with the patient seated, where the raw signal is filtered to identify features of interest, such as peaks 208 of the R-wave. A second signal recording 204 and a second filtered signal recording 206 may represent an additional raw signal with cardiac activity and filtered version of the additional raw signal, respectively, when the patient is walking. As can be seen in the examples of the second signal recording 204 and the second filtered signal recording 206, the patient walking/moving introduces noise to the signal recordings of cardiac activity as compared to the first signal recording 200 and the first filtered signal recording 202, where the patient is seated with no stimulation being applied. Accordingly, the noise depicted in the examples the second signal recording 204 and the second filtered signal recording 206 may need to be reduced or removed to obtain more accurate cardiac metrics or measurements for patient monitoring and/or supporting a closed-loop therapy system described herein.

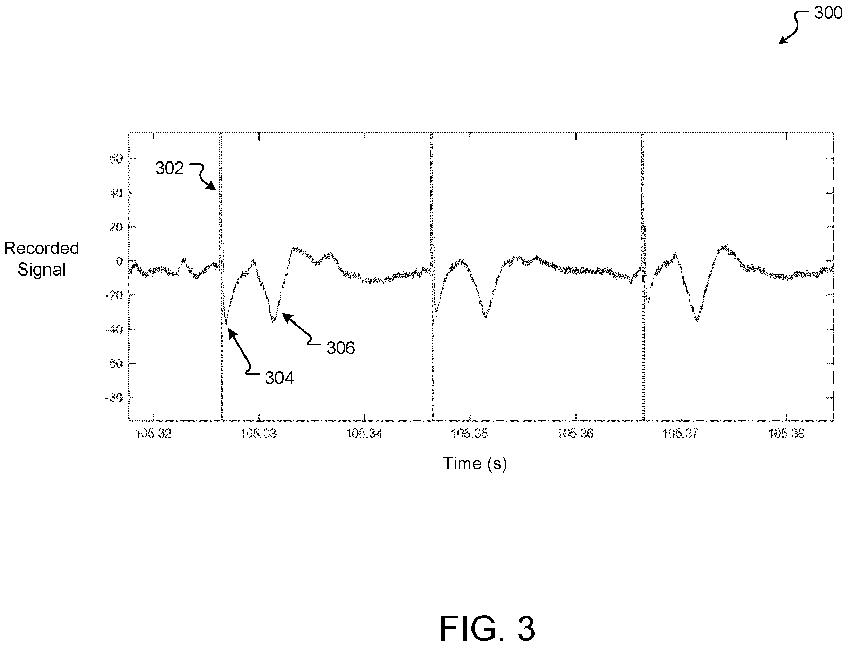

Fig. 3 is an example recorded signal 300 with noise present according to at least one embodiment of the present disclosure. In some examples, the example recorded signal 300 may be acquired by one or more aspects of Fig. 1. For example, the example recorded signal 300 may represent a physiological signal (e.g., cardiac electrogram signal(s)) that can be recorded by components of the system 100 (e.g., SCS leads, such as the leads 104 and/or the electrodes). However, the example recorded signal 300 may include one or more sources of noise, such as a stimulation artifact 302 (e.g., a response of the heart or body when the therapeutic electrical signal or stimulation is applied in the patient), an ECAP 304, and ECMAP activity 306. The sources of noise may obscure recordings of the cardiac signal (e.g., cardiac electrogram signal) on the SCS leads during electrical stimulation. In some examples, the sources of noise depicted in the example of Fig. 3 (e.g., the stimulation artifact 302, the ECAP 304, the ECMAP activity 306, or other evoked action potentials not explicitly listed or shown) may be responses that are time-synchronized to a stimulation pulse and often have characteristic relationships to the stimulation amplitude. For example, the sources of noise depicted in the example of Fig. 3 may occur based on a latency (e.g., 0-3ms) after the therapeutic electrical signal (e.g., stimulation) is applied in the patient, and characteristics of the sources of noise depicted in the example of Fig. 3 may depend or correspond to an amplitude of the therapeutic electrical signal. It is desirable to remove or reduce the sources of noise depicted in the example of Fig. 3 from recorded signals to correctly identify cardiac activity.

In some embodiments, for stimulation evoked signals (e.g., the stimulation artifact 302, the ECAP 304, the ECMAP activity 306, or other evoked action potentials not explicitly listed or shown), one method for removing the associated noise may include blanking of the samples at which the stimulation evoked signals are or may be present. For example, ECAPs on the spinal cord are usually present at latencies of 0-3 milliseconds (ms) after the therapeutic electrical signal (e.g., stimulation) is applied to the spinal cord (e.g., or to nearby nerves), so the corresponding time points according to the latencies may be blanked or processed using methods including template subtraction, spectral filtering, wavelet filtering, or a combination thereof. In some examples, the stimulation evoked signals (e.g., recorded signals that include cardiac activity) may be processed or blanked at the corresponding time points based at least in part on removing, attenuating, or replacing portions of the stimulation evoked signals at the corresponding time points with background activity with either hardware, firmware, and/or software methods. In some examples, the blanking or processing may be applied before, during, and/or after each pulse. Additionally, template subtraction may include subtracting a template comprising a best approximation of an artifact (e.g., movement artifact, noise, etc.) from the recorded signal.

Additionally or alternatively, growth curves may be collected to determine a threshold of activity, and signal processing may be performed for stimuli known to evoke large signals. For example, growth curves could be used to determine thresholds for ECAP activity, and the signal processing could be applied exclusively after stimulation amplitude exceeds a certain percentage of the threshold for ECAP activity (e.g., 10% above the threshold). The signal processing may also be used for ECMAPs or other evoked signals at relevant latencies after stimulation.

Another option for reducing stimulation artifacts is to cycle stimulation between ON phases and OFF phases. In one embodiment, such as DTM^{™} SCS, high frequency stimulation may be cycled while low frequency stimulation is continuous and/or cycled at lower ratios. In another embodiment, all stimuli are cycled. In the OFF phase of cycling, physiological signals may be collected so as to avoid contamination of stimulation artifacts. Additionally or alternatively, the physiological signals (e.g., cardiac electrogram signals, signals with cardiac activity, etc.) may be captured when a component used for applying the stimulation (e.g., therapeutic electrical signal) is off. For example, in the case of DTM^{™}, a high frequency component may be cycled separately from a low frequency component, and the physiological signals can be captured with more precision at times when the high frequency component is cycled off.

Additionally or alternatively, as noise is identified, "recording" parameters may be adjusted (e.g., in addition to or alternative to adjusting therapy parameters). That is, noise may be reduced from recorded signals (e.g., signals with cardiac activity as described herein) based on selecting electrodes configured for recording the signals such that noise is minimized. For example, a location of recording electrodes may be changed or adjusted, filtering parameters of a recording channel may be changed or adjusted (e.g., cutoff frequencies, bandwidth, etc.) based on a type of noise identified, noise floor of the recording channel may be adjusted, or a combination thereof. In some embodiments, the signal can be recorded using a noise filter with low energy/battery requirements that runs continuously with adequate noise reduction. In at least some embodiments, the noise filter can be switched to a noise filter that has a higher power consumption and improved filtering when the noise reaches an undesirable level or meets or exceeds a predetermined threshold. In other examples, changing or adjusting a location of recording electrodes for recording the signals with cardiac activity may include changing which device is recording the signals (e.g., an implanted or external device configured to record cardiac activity, the device 102 as described with reference to Fig. 1, etc.) to reduce noise based on spatial separation. Additionally or alternatively, different electrodes of SCS leads (e.g., the leads 104 as described with reference to Fig. 1) may be selected for recording the signals based on which electrodes experience less noise when noise is identified.

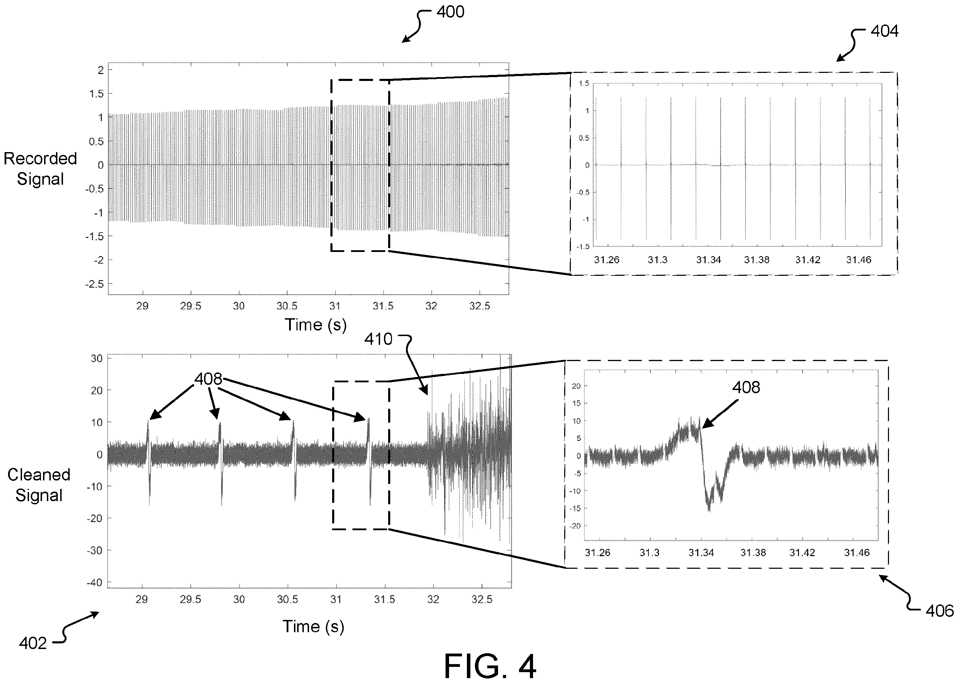

Fig. 4 is a set of example signal recordings (e.g., with cardiac activity) including electrical stimulation artifacts according to at least one embodiment of the present disclosure. The set of example signal recordings including electrical stimulation artifacts may be acquired by one or more aspects of Fig. 1. For example, the set of example signal recordings including electrical stimulation artifacts may represent physiological signals (e.g., cardiac electrogram signals) that can be recorded by components of the system 100 (e.g., SCS leads, such as the leads 104 and/or the electrodes). The set of example signal recordings including electrical stimulation artifacts depicted in the example of Fig. 4 may represent unwanted signals from stimulation, including electrical stimulation artifacts, that may be removed during or at specific times after the stimulus presentation so that accurate cardiac activity can be identified (e.g., for patient monitoring and/or supporting closed-loop neuromodulation). However, transient noises such as movement artifacts may obstruct the cardiac activity at times not correlated with stimulation.

A signal recording 400 may represent a signal with cardiac activity (e.g., cardiac electrogram signal) with electrical stimulation artifacts present. A filtered signal recording 402 may be a filtered version of the signal recording 400 with the electrical stimulation artifacts removed, such that one or more signals 408 indicating cardiac activity can be observed. However, even with the electrical stimulation artifacts removed, noise 410 may still be present (e.g., possibly caused by movement artifacts or other types of noise) that obstruct the cardiac signal. A first zoomed in portion 404 may represent a zoomed in section of the signal recording 400, and a second zoomed in portion 406 may represent a zoomed in section of the filtered signal recording 402, such that an enhanced view of one of the one or more signals 408 indicating cardiac activity can be observed.

Fig. 5 is a set of example signal recordings (e.g., with cardiac activity) for a patient while the patient is walking according to at least one embodiment of the present disclosure. In some examples, the set of example signal recordings for a patient while the patient is walking may be acquired by one or more aspects of Fig. 1. For example, the set of example cardiac signal recordings for a patient while the patient is walking may represent physiological signals (e.g., cardiac electrogram signals) that can be recorded by components of the system 100 (e.g., SCS leads, such as the leads 104 and/or the electrodes). A first signal recording 500 may represent a first raw signal with cardiac activity for the patient while the patient is walking, and a first filtered signal recording 502 may represent a filtered version of the first signal recording 500 for the patient while the patient is walking. A second signal recording 504 may represent a second raw signal with cardiac activity for the patient while the patient is walking, and a second filtered signal recording 506 may represent a filtered version of the second signal recording 504 for the patient while the patient is walking. The filtered signal recordings may be used to identify target components of signals with cardiac activity, such as the R-peak.

As illustrated in the first signal recording 500, the first filtered signal recording 502, the second signal recording 504, and the second filtered signal recording 506, walking (e.g., movement, movement artifacts, etc.) may degrade the quality of raw signals when analyzing cardiac activity (e.g., cardiac electrogram activity) on an SCS lead. In some embodiments, signal processing, such as filtering, may recover some of the cardiac activity (e.g., cardiac electrogram activity), such as depicted in the example of the first signal recording 500 and the first filtered signal recording 502. Additionally or alternatively, the signal processing (e.g., filtering) may obstruct the cardiac signal, resulting in missed or incorrect identification of features, such as depicted in the example of the second signal recording 504 and the second filtered signal recording 506.

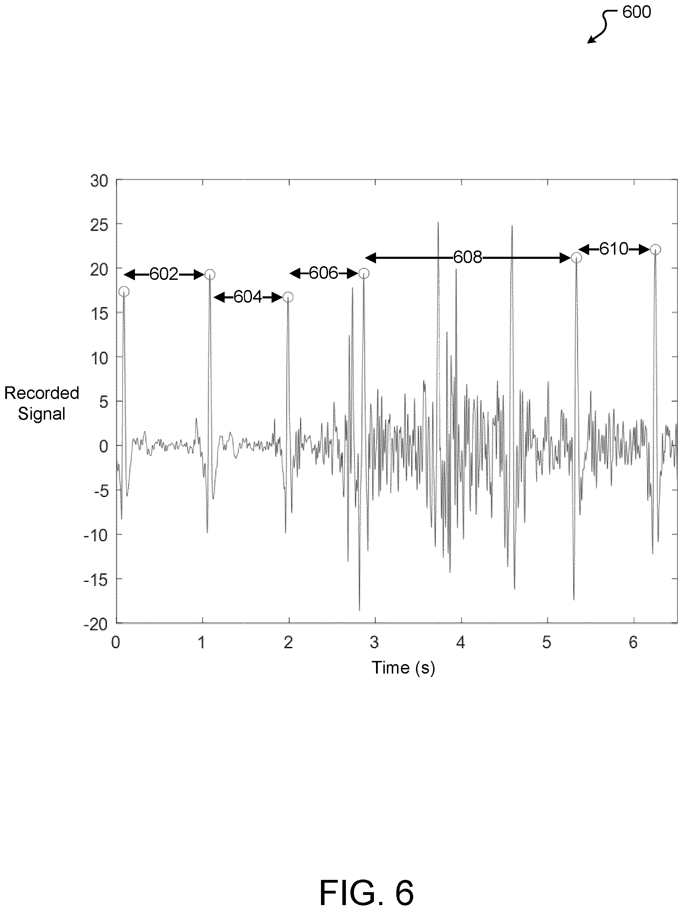

Fig. 6 is an example recorded, filtered signal 600 (e.g., with cardiac activity) with noise present according to at least one embodiment of the present disclosure. In some examples, the example recorded, filtered signal 600 may be acquired by one or more aspects of Fig. 1. For example, the example recorded, filtered signal 600 may represent a physiological signal (e.g., cardiac electrogram signal(s)) that can be recorded by components of the system 100 (e.g., SCS leads, such as the leads 104 and/or the electrodes). As depicted in the example of Fig. 6, the presence of noise may be indicated in a recorded signal based on missed heart beats or when consecutive R-wave peaks have an interval that is much larger than preceding intervals. For example, a first interval 602 may exist between a first R-wave peak and a second R-wave peak, a second interval 604 may exist between the second R-wave peak and a third R-wave peak, a third interval 606 may exist between the third R-wave peak and a fourth R-wave peak, a fourth interval 608 may exist between the fourth R-wave peak and a fifth R-wave peak, and a fifth interval 610 may exist between the fifth R-wave peak and a sixth R-wave peak. Accordingly, as can be seen, the fourth interval 608 is much longer or larger than the preceding intervals, which may indicate the presence of noise (e.g., to be removed from the recorded signal to accurately or correctly capture cardiac activity). In some embodiments, a defined threshold may be used to determine when a different R-R interval may represent the presence of noise. For example, if an R-R interval is more than 60% different than R-R intervals detected by preceding heart beats, the R-R interval may be determined to have a presence of noise. In such circumstances, cardiac electrogram measurements may be temporarily inhibited until the noise source resolves. Alternatively or additionally, in embodiments where the signal is recorded using a noise filter with low energy/battery requirements that runs continuously, the noise filter can be switched to a noise filter that has a higher power consumption and improved filtering when the R-R interval is more than 60% different than R-R intervals detected by preceding heart beats (which may indicate, for example, missed heart beats).

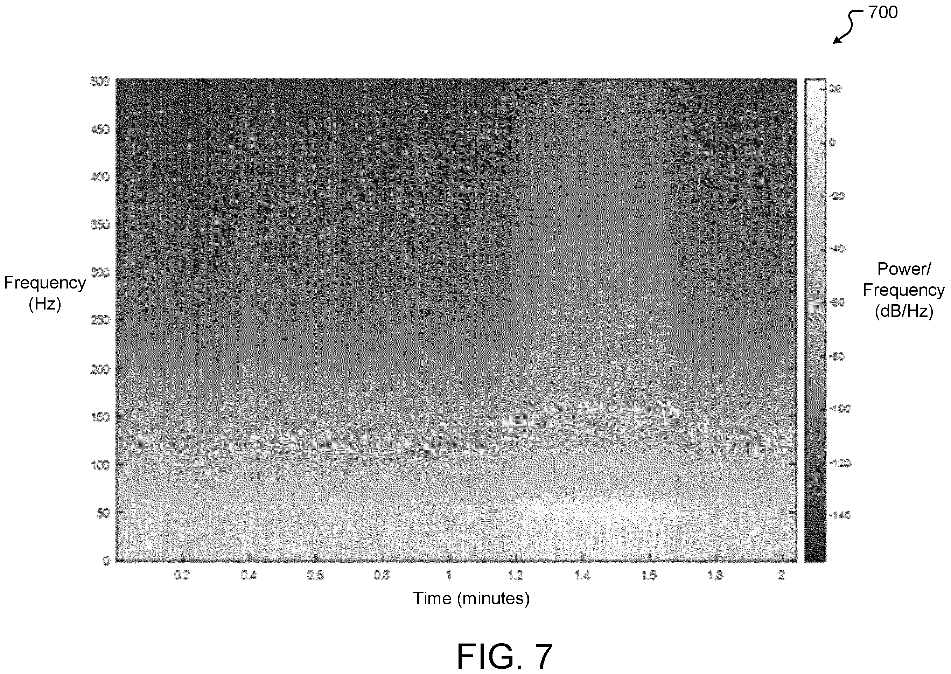

Fig. 7 is an example spectrogram 600 of filtered signals according to at least one embodiment of the present disclosure. In some examples, the example spectrogram 600 may be acquired by one or more aspects of Fig. 1. For example, the example spectrogram 600 may represent a spectrogram of filtered signals recorded on or by components of the system 100 (e.g., SCS leads, such as the leads 104 and/or the electrodes). As can be seen in the example of Fig. 6, the filtered signals in the example spectrogram 600 are contaminated at approximately 1.2-1.6 minutes (e.g., as illustrated by the distorted color and shading) by increased power at approximately 50-500Hz, indicating noise contaminating a recorded signal (e.g., with cardiac activity). More generally, as described herein, a presence of increased spectral power at or above a frequency threshold (e.g., 50Hz or greater) may indicate the presence of noise for a recorded signal. Accordingly, as described herein, it is desirable to remove or reduce such sources of noise from recorded signals to correctly capture cardiac activity for patient monitoring and/or supporting a closed-loop neuromodulation system that uses the recorded signals and captured cardiac activity to adjust therapy parameters for the neuromodulation system.

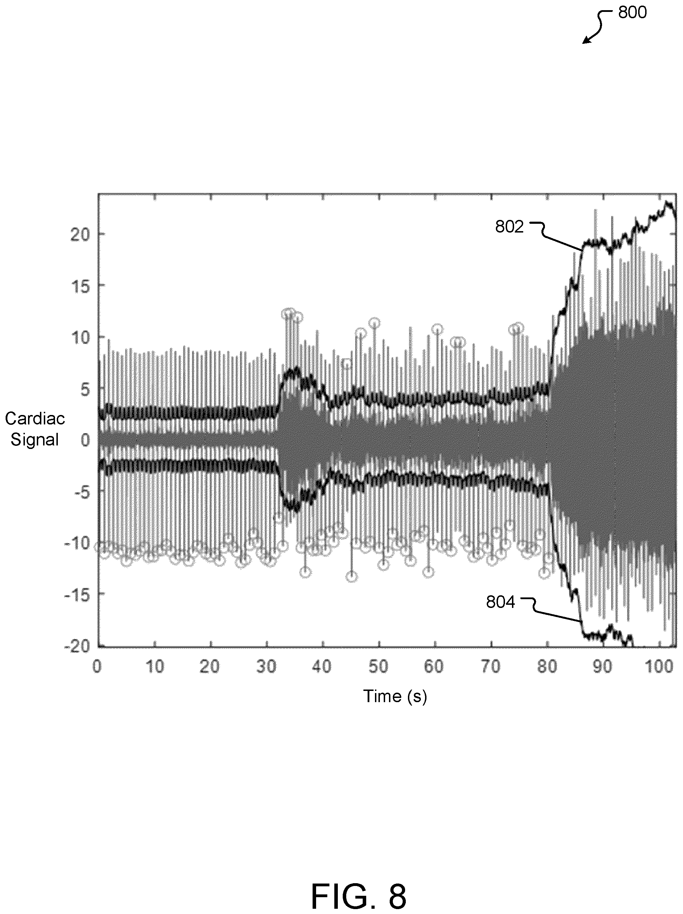

Fig. 8 is an example recorded signal 800 (e.g., with cardiac activity) with noise present according to at least one embodiment of the present disclosure. In some examples, the example recorded signal 800 may be acquired by one or more aspects of Fig. 1. For example, the example recorded signal 800 may represent a physiological signal (e.g., cardiac electrogram signal(s)) that can be recorded by components of the system 100 (e.g., SCS leads, such as the leads 104 and/or the electrodes). In some embodiments, the signal may is recorded using a noise filter with low energy/battery requirements that runs continuously. In such embodiments, one or more of a plurality of noise reduction algorithm with associated energy/battery needs and optimal algorithms may be selected based on the moving average noise and R-wave quality to optimize cardiac detection while minimizing battery drain.

As depicted in the example of Fig. 8, areas with excessive noise may be identified by tracking the magnitude of the envelope of the raw or processed signal via a moving average threshold (e.g., an upper bound threshold 802 and a lower bound threshold 804), where areas with low noise and good quality R-waves can be identified (e.g., the circled peaks and/or troughs) for capturing accurate cardiac metrics of the recorded cardiac signal that can be used to support a closed-loop neuromodulation system described herein. Additionally or alternatively, the moving average threshold(s) may be used to track the magnitude of the cardiac signal, and changes in the moving average threshold(s) may indicate areas with unacceptable noise (e.g., to be removed or reduced in the recorded cardiac signal). For example, the areas with low noise and good quality R-waves may be identified based on areas where the upper bound threshold 802 and/or the lower bound threshold 804 are fairly constant or flat (e.g., a change not exceeding more than 50% relative to the average threshold over the last minute).

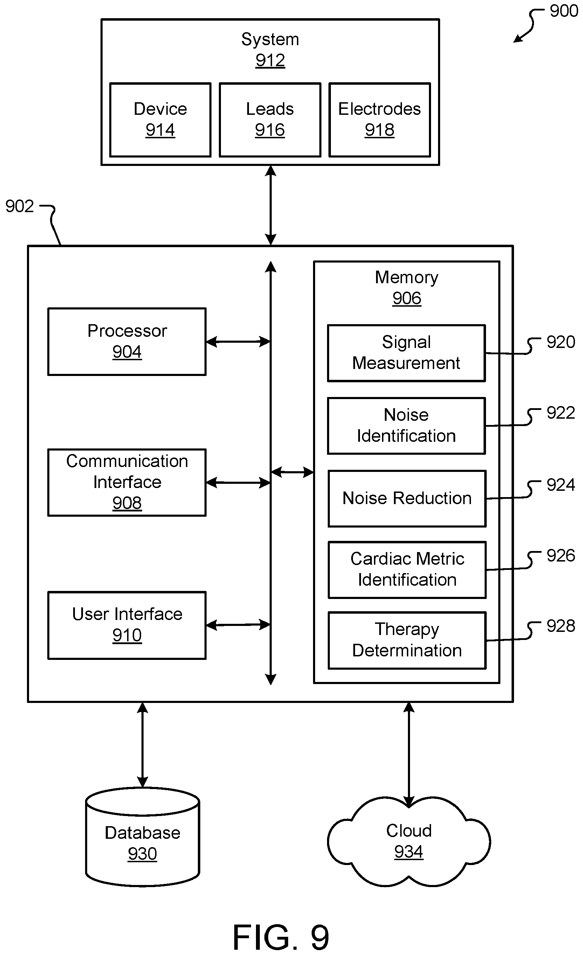

Turning to Fig. 9, a block diagram of a system 900 according to at least one embodiment of the present disclosure is shown. The system 900 may be used to identify and reduce noise from signals with cardiac activity and recordings for patient monitoring and/or to support closed-loop neuromodulation as described herein. In some examples, the system 900 may implement aspects of or may be implemented by aspects of Figs. 1-8 as described herein. For example, the system 900 may be used with a device 914, leads 916, and/or electrodes 918, and/or carry out one or more other aspects of one or more of the methods disclosed herein. The device 914 may represent an example of the device 102 or a component of the device 102 as described with reference to Fig. 1 (e.g., implantable pulse generator), where the leads 916 and the electrodes 918 may represent the leads 104 and corresponding electrodes/cuff electrodes as described with reference to Fig. 1. The system 900 comprises a computing device 902, a system 912, a database 930, and/or a cloud or other network 934. Systems according to other embodiments of the present disclosure may comprise more or fewer components than the system 900. For example, the system 900 may not include one or more components of the computing device 902, the database 930, and/or the cloud 934.

The system 912 may comprise the device 914, leads 916, and the electrodes 918. As previously described, the device 914 may be configured to generate a current (e.g., therapeutic electrical signal, stimulation signal, electrical stimulation signal, etc.), and the leads 916 and the electrodes 918 may comprise a plurality of electrodes configured to carry the current from the device 914 and apply the current to an anatomical element based on the electrodes being implanted on or near the anatomical element (e.g., stimulation target, such as the spinal cord 108 and/or nearby nerves to the spinal cord 108). In some examples, the device 914, leads 916, and electrodes 918 may be configured to measure a physiological response of the patient (e.g., prior to applying the current to the anatomical element, after the current is applied, etc.).

The system 912 may communicate with the computing device 902 to receive instructions such as instructions 924 for applying a current to the anatomical element and/or delivering the pharmacological agent to the anatomical element. The system 912 may also provide data (such as data received from an electrodes 918 capable of recording data), which may be used to optimize the electrodes 918 and/or to optimize parameters of the current generated by the device 914.

The computing device 902 comprises a processor 904, a memory 906, a communication interface 908, and a user interface 910. Computing devices according to other embodiments of the present disclosure may comprise more or fewer components than the computing device 902.

The processor 904 of the computing device 902 may be any processor described herein or any similar processor. The processor 904 may be configured to execute instructions 924 stored in the memory 906, which instructions may cause the processor 904 to carry out one or more computing steps utilizing or based on data received from the system 912, the database 930, and/or the cloud 934.

The memory 906 may be or comprise RAM, DRAM, SDRAM, other solid-state memory, any memory described herein, or any other tangible, non-transitory memory for storing computer-readable data and/or instructions. The memory 906 may store information or data useful for completing, for example, any steps of the methods 1000, 1100, and/or 1200 described herein, or of any other methods. The memory 906 may store, for example, instructions and/or machine learning models that support one or more functions of the system 912. For instance, the memory 906 may store content (e.g., instructions and/or machine learning models) that, when executed by the processor 904, enable a signal measurement 920, a noise identification 922, a noise reduction 924, a cardiac metric identification 926, and a therapy determination 928.

The signal measurement 920 enables the processor 904 to measure (e.g., via the leads 916, one or more of the electrodes 918, etc.) one or more signals including cardiac activity from the patient. For example, as described with reference to Figs. 1-8, the signals may include cardiac electrogram signals and may include cardiac-derived metrics such as HR, HRV, respiration, etc. In some examples, the one or more signals may be measured when the patient is stationary or in a specific position. In some embodiments, instructions stored in the memory 906 may cause the processor 904 to perform the cardiac signal measurement 920 as described.

The noise identification 922 enables the processor 904 to identify one or more sources of noise that are distorting the one or more signals. For example, as described with reference to Figs. 1-8, the one or more sources of noise are caused by the patient walking or moving, a stimulation artifact, environmental noise, ECAP activity, ECMAP activity, or a combination thereof. The noise identification 922 also enables the processor 904 to identify time points when the one or more signals have been corrupted by the one or more sources of noise. For example, the time points may be identified based at least in part on time intervals when a given R-R interval is non-physiological and/or differs from R-R intervals detected for preceding heartbeats by a defined threshold (e.g., as illustrated and described with reference to Fig. 6), when a presence of increased spectral power of greater than a frequency threshold is identified (e.g., as illustrated and described with reference to Fig. 7), when changes in a magnitude threshold for the therapeutic electrical signal are identified (e.g., as illustrated and described with reference to Fig. 8), when movement is detected for the patient, or a combination thereof. In some embodiments, instructions stored in the memory 906 may cause the processor 904 to perform the noise identification 922 as described.

The noise reduction 924 enables the processor 904 to reduce the one or more sources of noise from the one or more signals. For example, as described with reference to Figs. 1-8, the one or more sources of noise may be identified and reduced or removed from the signals. The noise reduction 924 also enables the processor 904 to process one or more portions of the one or more signals, the one or more portions corresponding to time points when the one or more sources of noise are expected to occur after the therapeutic electrical signal is applied to the anatomical element. For example, the one or more portions of the one or more signals may be blanked before, during, and/or after individual pulses of the therapeutic electrical signal are applied to the anatomical element (e.g., removing, attenuating, or replacing portions of the one or more signals at corresponding time points with background activity with either hardware, firmware, and/or software methods). In some examples, the one or more portions of the one or more signals are processed based at least in part on a template subtraction method (i.e., the contribution of an artifact is approximated as a template before being subtracted from the one or more signals), spectral filtering, wavelet filtering, common-mode signal rejection, principal component analysis, or a combination thereof.

Additionally or alternatively, the noise reduction 924 enables the processor 904 to generate one or more growth curves based at least in part on applying the therapeutic electrical signal to the anatomical element, determine an evoked action potential threshold based at least in part on the one or more growth curves, and perform signal processing for stimuli that evoke signals larger than the evoked action potential threshold to measure the one or more signals, wherein the one or more sources of noise are reduced based at least in part on performing the signal processing for stimuli that evoke signals larger than the evoked action potential threshold. In some examples, the stimuli that evoke signals larger than the evoked action potential threshold comprise stimulation amplitudes for the therapeutic electrical signal.

In some embodiments, the noise reduction 924 also enables the processor 904 to cycle one or more parameters used for applying the therapeutic electrical signal to the anatomical element between 'on' phases and 'off' phases, wherein the one or more signals are measured during the `off phases. For example, the 'on' phases comprise applying the therapeutic electrical signal to the anatomical element using a combination or high frequency and/or low frequency stimulations and/or high and/or low amplitudes, and the 'off' phases comprise applying the therapeutic electrical signal to the anatomical element using low frequency stimulations, lower ratios of cycling, low amplitudes, or a combination thereof. Additionally or alternatively, the therapy may be configured or timed based on cardiac pulses of the patient. For example, therapy pulses may be delivered during a cardiac refractory period and/or in between cardiac pulses to reduce noise from the one or more measured and/or recorded signals with cardiac activity.

In some embodiments, the noise reduction 924 also enables the processor 904 to filter the one or more signals to reduce the one or more sources of noise. Additionally or alternatively, the noise reduction 924 also enables the processor 904 to remove portions of the one or more signals corresponding to the identified time points when the one or more signals have been corrupted by the one or more sources of noise to reduce the one or more sources of noise from the one or more signals. In some embodiments, instructions stored in the memory 906 may cause the processor 904 to perform the noise reduction 924 as described.

The cardiac metric identification 926 enables the processor 904 to determine one or more aggregate cardiac-derived metrics and/or save processed cardiac data based at least in part on the one or more signals with the one or more sources of noise reduced. For example, the cardiac-derived metrics may be aggregated for times of interest and/or for "best" signals (e.g., low-noise conditions, when the patient is not moving, etc.). In some embodiments, instructions stored in the memory 906 may cause the processor 904 to perform the cardiac metric identification 926 as described.

The therapy determination 928 enables the processor 904 to determine one or more parameters for applying the therapeutic electrical signal to the anatomical element based at least in part on the one or more aggregate cardiac-derived metrics and/or saved processed cardiac data. For example, as described with reference to Figs. 1-8, the cardiac-derived measures (e.g., cardiac signal with the sources of noise removed or reduced) may be used to determine and/or adjust one or more parameters for applying the therapeutic electrical signal to the anatomical element (e.g., spinal cord 108 and/or nearby nerves) to optimally provide pain treatment for the patient (e.g., as part of a closed-loop neuromodulation therapy). As an example, chronic pain state has been correlated with an increase in baseline HR and/or decrease in HRV (e.g., derived from cardiac electrogram signal(s)), and the SCS therapy provided can bring these biomarkers closer to normal range to cause or correspond with increased pain relief by determining and/or adjusting the one or more parameters for applying the therapeutic electrical signal. Additionally or alternatively, the therapy determination 928 enables the processor 904 to output the one or more aggregate cardiac-derived metrics and/or save processed cardiac data (e.g., for patient monitoring, for a physician and/or the patient to determine or adjust the one or more parameters, etc.). In some embodiments, instructions stored in the memory 906 may cause the processor 904 to perform the therapy determination 928 as described.

Content stored in the memory 906, if provided as in instruction, may, in some embodiments, be organized into one or more applications, modules, packages, layers, or engines. Alternatively or additionally, the memory 906 may store other types of content or data (e.g., machine learning models, artificial neural networks, deep neural networks, etc.) that can be processed by the processor 904 to carry out the various method and features described herein. Thus, although various contents of memory 906 may be described as instructions, it should be appreciated that functionality described herein can be achieved through use of instructions, algorithms, and/or machine learning models. The data, algorithms, and/or instructions may cause the processor 904 to manipulate data stored in the memory 906 and/or received from or via the system 912, the database 930, and/or the cloud 934.

The computing device 902 may also comprise a communication interface 908. The communication interface 908 may be used for receiving data (for example, data from the electrodes 918 capable of recording data) or other information from an external source (such as the system 912, the database 930, the cloud 934, and/or any other system or component not part of the system 900), and/or for transmitting instructions, images, or other information to an external system or device (e.g., another computing device 902, the system 912, the database 930, the cloud 934, and/or any other system or component not part of the system 900). The communication interface 908 may comprise one or more wired interfaces (e.g., a USB port, an Ethernet port, a Firewire port) and/or one or more wireless transceivers or interfaces (configured, for example, to transmit and/or receive information via one or more wireless communication protocols such as 902.11a/b/g/n, Bluetooth, NFC, ZigBee, and so forth). In some embodiments, the communication interface 908 may be useful for enabling the device 902 to communicate with one or more other processors 904 or computing devices 902, whether to reduce the time needed to accomplish a computing-intensive task or for any other reason.

The computing device 902 may also comprise one or more user interfaces 910. The user interface 910 may be or comprise a keyboard, mouse, trackball, monitor, television, screen, touchscreen, and/or any other device for receiving information from a user and/or for providing information to a user. The user interface 910 may be used, for example, to receive a user selection or other user input regarding any step of any method described herein. Notwithstanding the foregoing, any required input for any step of any method described herein may be generated automatically by the system 900 (e.g., by the processor 904 or another component of the system 900) or received by the system 900 from a source external to the system 900. In some embodiments, the user interface 910 may be useful to allow a surgeon or other user to modify instructions to be executed by the processor 904 according to one or more embodiments of the present disclosure, and/or to modify or adjust a setting of other information displayed on the user interface 910 or corresponding thereto.

Although the user interface 910 is shown as part of the computing device 902, in some embodiments, the computing device 902 may utilize a user interface 910 that is housed separately from one or more remaining components of the computing device 902. In some embodiments, the user interface 910 may be located proximate one or more other components of the computing device 902, while in other embodiments, the user interface 910 may be located remotely from one or more other components of the computer device 902.

Though not shown, the system 900 may include a controller, though in some embodiments the system 900 may not include the controller. The controller may be an electronic, a mechanical, or an electro-mechanical controller. The controller may comprise or may be any processor described herein. The controller may comprise a memory storing instructions for executing any of the functions or methods described herein as being carried out by the controller. In some embodiments, the controller may be configured to simply convert signals received from the computing device 902 (e.g., via a communication interface 109) into commands for operating the system 912 (and more specifically, for actuating the device 914 and/or the electrodes 918). In other embodiments, the controller may be configured to process and/or convert signals received from the system 912. Further, the controller may receive signals from one or more sources (e.g., the system 912) and may output signals to one or more sources.

The database 930 may store information such as patient data, results of a stimulation and/or blocking procedure, stimulation and/or blocking parameters, current parameters, electrode parameters, etc. The database 930 may be configured to provide any such information to the computing device 902 or to any other device of the system 900 or external to the system 900, whether directly or via the cloud 934. In some embodiments, the database 930 may be or comprise part of a hospital image storage system, such as a picture archiving and communication system (PACS), a health information system (HIS), and/or another system for collecting, storing, managing, and/or transmitting electronic medical records.

The cloud 934 may be or represent the Internet or any other wide area network. The computing device 902 may be connected to the cloud 934 via the communication interface 908, using a wired connection, a wireless connection, or both. In some embodiments, the computing device 902 may communicate with the database 930 and/or an external device (e.g., a computing device) via the cloud 934.

The system 900 or similar systems may be used, for example, to carry out one or more aspects of any of the methods 1000, 1100, and/or 1200 as described herein. The system 900 or similar systems may also be used for other purposes.

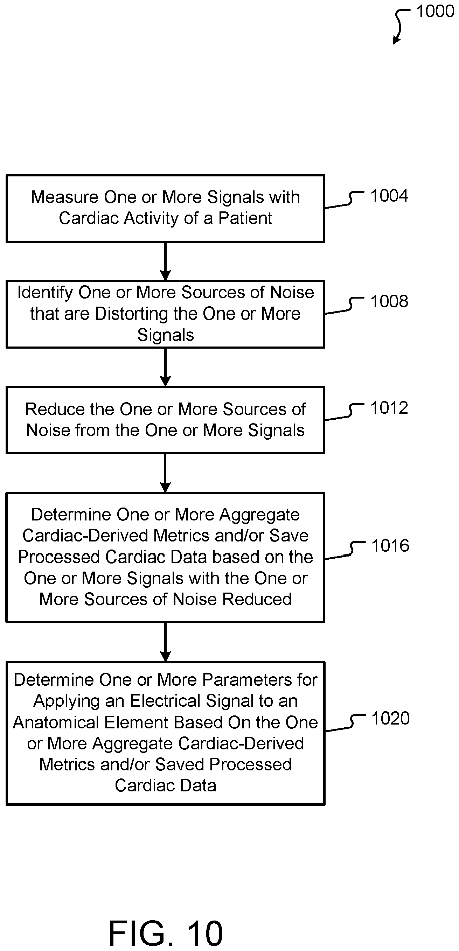

Fig. 10 depicts a method 1000 that may be used, for example, to identify and reduce noise present in recorded signals with cardiac activity to more accurately capture the cardiac activity.

The method 1000 (and/or one or more steps thereof) may be carried out or otherwise performed, for example, by at least one processor. The at least one processor may be the same as or similar to the processor(s) 904 of the computing device 902 described above. The at least one processor may be the same as or similar to the processor(s) of the device 104, 914 described above. The at least one processor may be part of the device 104, 914 (such as an implantable pulse generator) or part of a control unit in communication with the device 104, 914. A processor other than any processor described herein may also be used to execute the method 1000. The at least one processor may perform the method 1000 by executing elements stored in a memory such as the memory 906. The elements stored in the memory and executed by the processor may cause the processor to execute one or more steps of a function as shown in method 1000. One or more portions of a method 1000 may be performed by the processor executing any of the contents of memory, such as a signal measurement 920, a noise identification 922, a noise reduction 924, a cardiac metric identification 926, and/or a therapy determination 928.

The method 1000 comprises measuring, via one or more of a plurality of electrodes (e.g., and/or SCS leads as described herein), one or more signals with cardiac activity of the patient (step 1004). For example, the one or more signals with cardiac activity may include cardiac electrogram signals and may be used to collect cardiac-derived metrics, including HR, HRV, respiration, etc. In some examples, the one or more signals with cardiac activity may be measured when the patient is stationary or in a specific position (e.g., to minimize an impact of possible noise and/or movement artifacts from impacting collecting the cardiac-derived metrics from the signals).

The method 1000 also comprises identifying one or more sources of noise that are distorting the one or more signals with cardiac activity (step 1008). In some embodiments, the one or more sources of noise are caused by the patient walking or moving, a stimulation artifact, environmental noise, ECAP activity, ECMAP activity, or a combination thereof.

The method 1000 also comprises reducing the one or more sources of noise from the one or more signals with cardiac activity (step 1012). In some embodiments, the one or more sources of noise may be reduced from the one or more signals based on processing one or more portions of the one or more signals, where the one or more portions correspond to time points when the one or more sources of noise are expected to occur after a therapeutic electrical signal is applied to an anatomical element of a patient. For example, the one or more portions of the one or more cardiac signals may be blanked after individual pulses of the therapeutic electrical signal are applied to the anatomical element. In some examples, the one or more portions of the one or more signals are processed based at least in part on a template subtraction method, spectral filtering, wavelet filtering, or a combination thereof.

Additionally or alternatively, the one or more sources of noise may be reduced from the one or more signals with cardiac activity based on cycling one or more parameters used for applying the therapeutic electrical signal to the anatomical element between 'on' phases and 'off' phases, wherein the one or more signals are measured during the 'off' phases. For example, the 'on' phases comprise applying the therapeutic electrical signal to the anatomical element using high frequency stimulations and/or high amplitudes, and the 'off' phases comprise applying the therapeutic electrical signal to the anatomical element using low frequency stimulations, lower ratios of cycling, low amplitudes, or a combination thereof. In some embodiments, the one or more sources of noise may be reduced from the one or more signals based on filtering the one or more signals to reduce the one or more sources of noise (e.g., as illustrated and described with reference to Fig. 5).

The method 1000 also comprises determining one or more aggregate cardiac-derived metrics and/or save processed cardiac data based at least in part on the one or more signals (e.g., with cardiac activity) with the one or more sources of noise reduced (step 1016). For example, the cardiac-derived measures and/or saved processed cardiac data may comprise HR, HRV, respiration, etc. In some examples, the cardiac-derived metrics may be aggregated over specific times or conditions, such as low-noise conditions, when the patient is determined to not be moving, etc.

The method 1000 also comprises determining one or more parameters for applying the therapeutic electrical signal to the anatomical element based at least in part on the one or more aggregate cardiac-derived metrics and/or save processed cardiac data (step 1020). For example, as described with reference to Figs. 1-9, the cardiac-derived measures (e.g., HR, HRV, respiration, etc.) may be used to determine and/or adjust one or more parameters for applying the therapeutic electrical signal to the anatomical element (e.g., spinal cord 108 and/or nearby nerves) to optimally provide pain relief for the patient (e.g., as part of a closed-loop neuromodulation therapy). As an example, chronic pain state has been correlated with an increase in baseline HR and/or decrease in HRV (e.g., metrics derived from cardiac electrogram signal(s)), and the SCS therapy provided can bring these biomarkers closer to normal range to cause or correspond with increased pain relief by determining and/or adjusting the one or more parameters for applying the therapeutic electrical signal (e.g., based on an algorithm as described herein). For example, an amplitude of the therapeutic electrical signal may be increased, cycling of the therapeutic electrical signal may be adjusted, the electrodes and/or SCS leads may be adjusted, and/or a different adjustment may be made to provide better pain relief for the patient based on the signals with the sources of noise removed or reduced.

Additionally or alternatively, the one or more aggregate cardiac-derived metrics and/or save processed cardiac data may be output to an operator, physician, the patient, or another user (e.g., via a user interface). For example, the one or more aggregate cardiac-derived metrics and/or save processed cardiac data may be used for patient monitoring, to determine the one or more parameters for applying the therapeutic electrical signal to the anatomical element, or a combination thereof.

The present disclosure encompasses embodiments of the method 1000 that comprise more or fewer steps than those described above, and/or one or more steps that are different than the steps described above.

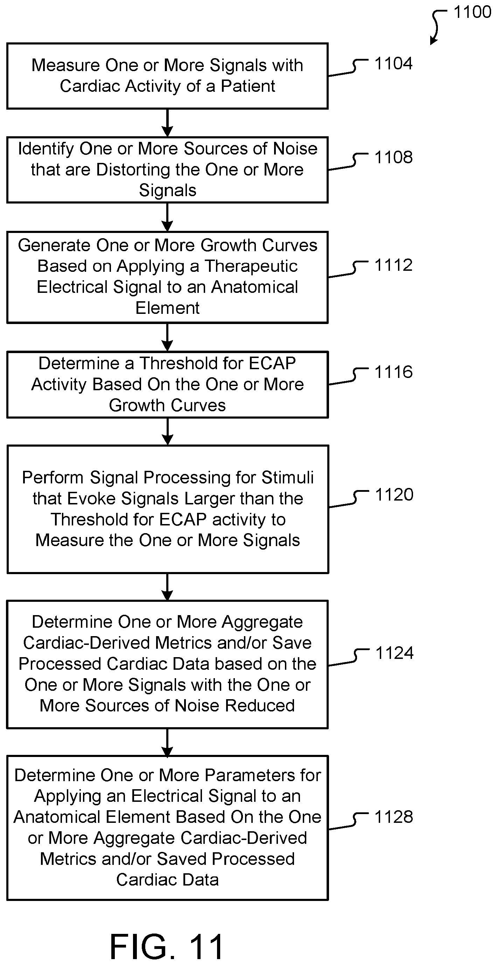

Fig. 11 depicts a method 1100 that may be used, for example, to perform selective signal processing to remove or reduce noise from recorded signals to more accurately determine cardiac-derived metrics.

The method 1100 (and/or one or more steps thereof) may be carried out or otherwise performed, for example, by at least one processor. The at least one processor may be the same as or similar to the processor(s) 904 of the computing device 902 described above. The at least one processor may be the same as or similar to the processor(s) of the device 104, 914 described above. The at least one processor may be part of the device 104, 914 (such as an implantable pulse generator) or part of a control unit in communication with the device 104, 914. A processor other than any processor described herein may also be used to execute the method 1100. The at least one processor may perform the method 1100 by executing elements stored in a memory such as the memory 906. The elements stored in the memory and executed by the processor may cause the processor to execute one or more steps of a function as shown in method 1100. One or more portions of a method 1100 may be performed by the processor executing any of the contents of memory, such as a signal measurement 920, a noise identification 922, a noise reduction 924, a cardiac metric identification 926, and/or a therapy determination 928.

The method 1100 comprises measuring, via one or more of a plurality of electrodes (e.g., and/or SCS leads as described herein), one or more signals with cardiac activity of the patient (step 1104). Step 1104 may implement similar aspects of step 1004 as described with reference to Fig. 10. The method 1100 also comprises identifying one or more sources of noise that are distorting the one or more signals with cardiac activity (step 1108). Step 1108 may implement similar aspects of step 1008 as described with reference to Fig. 10.

The method 1100 also comprises generating one or more growth curves based on applying a therapeutic electrical signal to an anatomical element (step 1112). The method 1100 also comprises determining a threshold for ECAP activity based on the one or more growth curves (step 1116). The method 1100 also comprises performing signal processing for stimuli that evoke signals larger than the threshold for ECAP activity to measure the one or more signals, wherein the one or more sources of noise are reduced based on performing the signal processing for stimuli that evoke signals larger than this threshold for ECAP activity (step 1120). In some examples, the stimuli that evoke signals larger than the threshold may include stimulation amplitudes for the therapeutic electrical signal. In some examples, the threshold may be an evoked action potential threshold, where an ECAP is first observed in the signal.

The method 1100 also comprises determining one or more aggregate cardiac-derived metrics and/or save processed cardiac data based at least in part on the one or more signals (e.g., with cardiac activity) with the one or more sources of noise reduced (step 1124). Step 1124 may implement similar aspects of step 1016 as described with reference to Fig. 10. In some embodiments, the cardiac-derived metrics may be analyzed over periods where evoked action potentials (e.g., ECAPs) are smaller than the determined evoked action potential threshold.

The method 1100 also comprises determining one or more parameters for applying the therapeutic electrical signal to the anatomical element based at least in part on the one or more aggregate cardiac-derived metrics and/or saved processed cardiac data (step 1128). Step 1128 may implement similar aspects of step 1016 as described with reference to Fig. 10.

The present disclosure encompasses embodiments of the method 1100 that comprise more or fewer steps than those described above, and/or one or more steps that are different than the steps described above.

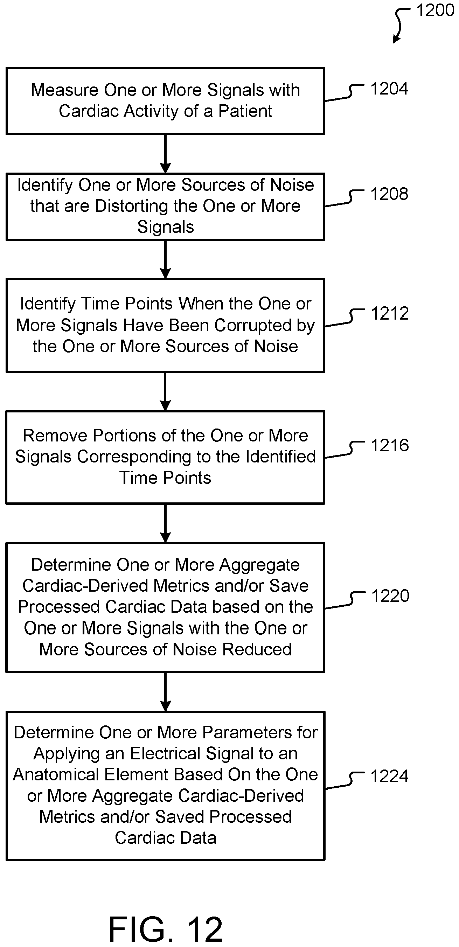

Fig. 12 depicts a method 1200 that may be used, for example, to identify and remove corrupted portions of recorded signals with cardiac activity.

The method 1200 (and/or one or more steps thereof) may be carried out or otherwise performed, for example, by at least one processor. The at least one processor may be the same as or similar to the processor(s) 904 of the computing device 902 described above. The at least one processor may be the same as or similar to the processor(s) of the device 104, 914 described above. The at least one processor may be part of the device 104, 914 (such as an implantable pulse generator) or part of a control unit in communication with the device 104, 914. A processor other than any processor described herein may also be used to execute the method 1200. The at least one processor may perform the method 1200 by executing elements stored in a memory such as the memory 906. The elements stored in memory and executed by the processor may cause the processor to execute one or more steps of a function as shown in method 1200. One or more portions of a method 1200 may be performed by the processor executing any of the contents of memory, such as a signal measurement 920, a noise identification 922, a noise reduction 924, a cardiac metric identification 926, and/or a therapy determination 928.

The method 1200 comprises measuring, via one or more of a plurality of electrodes (e.g., and/or SCS leads as described herein), one or more signals with cardiac activity of the patient (step 1204). Step 1204 may implement similar aspect of steps 1004 and 1104 as described with reference to Figs. 10 and 11. The method 1200 also identifying one or more sources of noise that are distorting the one or more signals with cardiac activity (step 1208). Step 1208 may implement similar aspect of steps 1008 and 1108 as described with reference to Figs. 10 and 11.

The method 1200 also comprises identifying time points when the one or more signals with cardiac activity have been corrupted by the one or more sources of noise (step 1212). For example, the time points may be identified based at least in part on time intervals when a given R-R interval is non-physiological and/or differs from R-R intervals detected for preceding heartbeats by a defined threshold (e.g., as illustrated and described with reference to Fig. 6), when a presence of increased spectral power of greater than a frequency threshold is identified (e.g., as illustrated and described with reference to Fig. 7), when changes in a magnitude threshold for the therapeutic electrical signal are identified (e.g., as illustrated and described with reference to Fig. 8), when movement is detected for the patient, or a combination thereof.

The method 1200 also comprises removing portions of the one or more signals with cardiac activity corresponding to the identified time points to reduce the one or more sources of noise from the one or more signals with cardiac activity (step 1216).

The method 1200 also comprises determining one or more aggregate cardiac-derived metrics and/or save processed cardiac data based at least in part on the one or more signals (e.g., with cardiac activity) with the one or more sources of noise reduced (step 1220). Step 1220 may implement similar aspects of steps 1016 and 1124 as described with reference to Figs. 10 and 11.

The method 1200 also comprises determining one or more parameters for applying the therapeutic electrical signal to the anatomical element based at least in part on one or more aggregate cardiac-derived metrics and/or saved processed cardiac data (step 1224). Step 1224 may implement similar aspects of step 1020 and 1128 as described with reference to Figs. 10 and 11.

The present disclosure encompasses embodiments of the method 1200 that comprise more or fewer steps than those described above, and/or one or more steps that are different than the steps described above.

As noted above, the present disclosure encompasses methods with fewer than all of the steps identified in Figs. 10, 11, and 12 (and the corresponding description of the methods 1000, 1100, and 1200), as well as methods that include additional steps beyond those identified in Figs. 10, 11, and 12 (and the corresponding description of the methods 1000, 1100, and 1200). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

A system is provided for identifying and reducing noise in a therapeutic procedure. For example, the system may be configured to measure one or more signals with cardiac activity. The system may be configured to then identify one or more sources of noise that are distorting the one or more signals with cardiac activity and may reduce the one or more sources of noise from the one or more signals with cardiac activity. Subsequently, the system may be configured to determine one or more aggregate cardiac-derived metrics and/or save processed cardiac data based on the one or more signals with cardiac activity with the one or more sources of noise reduced. Additionally, the system may be configured to determine one or more parameters for applying an electrical signal to an anatomical element of a patient based on the one or more aggregate cardiac-derived metrics and/or saved processed cardiac data.

Moreover, the following clauses relate to the present disclosure:
1. A system for identifying and reducing noise in a therapeutic procedure, comprising:
   a pulse generator configured to generate a therapeutic electrical signal;
   one or more leads in communication with the pulse generator and configured to transmit the therapeutic electrical signal to a plurality of electrodes;
   the plurality of electrodes in communication with the one or more leads, the plurality of electrodes configured to apply the therapeutic electrical signal to an anatomical element of a patient and configured to measure a physiological response;
   a processor; and
   a memory storing data for processing by the processor, the data, when processed, causes the processor to:
      measure, via one or more of the plurality of electrodes, one or more signals with cardiac activity of the patient;
      identify one or more sources of noise that are distorting the one or more signals with cardiac activity;
      reduce the one or more sources of noise from the one or more signals with cardiac activity;
      determine one or more aggregate cardiac-derived metrics and/or save processed cardiac data based at least in part on the one or more signals with cardiac activity with the one or more sources of noise reduced; and
      determine one or more parameters for applying the therapeutic electrical signal to the anatomical element based at least in part on the one or more aggregate cardiac-derived metrics and/or saved processed cardiac data.
2. The system of clause 1, wherein the memory stores further data for processing by the processor that, when processed, causes the processor to:
   process one or more portions of the one or more signals with cardiac activity, the one or more portions corresponding to time points when the one or more sources of noise are expected to occur after the therapeutic electrical signal is applied to the anatomical element.
3. The system of clause 2, wherein the one or more portions of the one or more signals with cardiac activity are blanked immediately before, during, and/or after individual pulses of the therapeutic electrical signal are applied to the anatomical element.
4. The system of clause 2, wherein the one or more portions of the one or more signals with cardiac activity are processed based at least in part on a template subtraction method, spectral filtering, wavelet filtering, or a combination thereof.
5. The system of clause 1, wherein the memory stores further data for processing by the processor that, when processed, causes the processor to:
   generate one or more growth curves based at least in part on applying the therapeutic electrical signal to the anatomical element;
   determine a threshold based at least in part on the one or more growth curves, wherein the one or more aggregate cardiac-derived metrics are collected when evoked signals are below the determined threshold; and
   perform signal processing for stimuli that evoke signals larger than the threshold to measure the one or more signals with cardiac activity, wherein the one or more sources of noise are reduced based at least in part on performing the signal processing for stimuli that evoke signals larger than the threshold.
6. The system of clause 5, wherein the stimuli that evoke signals larger than the threshold comprise stimulation amplitudes for added processing.
7. The system of clause 1, wherein the data stored in the memory that, when processed causes the processor to reduce the one or more sources of noise from the one or more signals with cardiac activity further causes the system to:
   cycle one or more parameters used for applying the therapeutic electrical signal to the anatomical element and/or one or more parameters for measuring the one or more signals with cardiac activity between 'on' phases and 'off' phases, wherein the one or more signals with cardiac activity are measured during the 'off' phases.
8. The system of clause 7, wherein the 'on' phases comprise applying the therapeutic electrical signal to the anatomical element using a combination or high frequency and/or low frequency stimulations and/or high and/or low amplitudes, and the 'off' phases comprise applying the therapeutic electrical signal to the anatomical element using low frequency stimulations, lower ratios of cycling, low amplitudes, or a combination thereof.
9. The system of clause 1, wherein the data stored in the memory that, when processed causes the processor to reduce the one or more sources of noise from the one or more signals with cardiac activity further causes the system to:
   filter the one or more signals with cardiac activity to reduce the one or more sources of noise.
10. The system of clause 1, wherein the data stored in the memory that, when processed causes the processor to reduce the one or more sources of noise from the one or more signals with cardiac activity further causes the system to:
   identify time points when the one or more signals with cardiac activity have been corrupted by the one or more sources of noise; and
   remove portions of the one or more signals with cardiac activity corresponding to the identified time points to reduce the one or more sources of noise from the one or more signals with cardiac activity.
11. The system of clause 10, wherein the time points are identified based at least in part on time intervals when a given R-R interval is non-physiological and/or differs from R-R intervals detected for preceding heartbeats by a defined threshold, when a presence of increased spectral power of greater than a frequency threshold is identified, when changes in a magnitude threshold for the one or more signals with cardiac activity are identified, when movement is detected for the patient, or a combination thereof.
12. The system of clause 1, wherein the one or more signals with cardiac activity of the patient are measured using a continuously running noise filter, wherein the noise filter is adjusted when the one or more sources of noise meet or exceed a predetermined threshold, wherein the adjusted noise filter uses more power than the non-adjusted noise filter.
13. The system of clause 1, wherein the memory stores further data for processing by the processor that, when processed, causes the processor to:
   output, via a user interface, the one or more aggregate cardiac-derived metrics and/or the saved processed cardiac data, wherein the one or more parameters for applying the therapeutic electrical signal to the anatomical element are determined based at least in part on outputting the one or more aggregate cardiac-derived metrics and/or the saved processed cardiac data.
14. The system of clause 1, wherein the one or more signals with cardiac activity are measured when the patient is stationary or in a specific position.
15. The system of clause 1, wherein the one or more sources of noise are caused by the patient walking or moving, a stimulation artifact, environmental noise, evoked compound action potential activity, evoked compound muscle action potential activity, or a combination thereof.
16. A system for identifying and reducing noise in a therapeutic procedure, comprising:
   a processor; and
   a memory storing data for processing by the processor, the data, when processed, causes the processor to:
      measure, via one or more of a plurality of electrodes, one or more signals with cardiac activity of a patient;
      identify one or more sources of noise that are distorting the one or more signals with cardiac activity;
      reduce the one or more sources of noise from the one or more signals with cardiac activity;
      determine one or more aggregate cardiac-derived metrics and/or save processed cardiac data based at least in part on the one or more signals with cardiac activity with the one or more sources of noise reduced; and
      determine one or more parameters for applying a therapeutic electrical signal to an anatomical element based at least in part on the one or more aggregate cardiac-derived metrics and/or saved processed cardiac data.
17. The system of clause 16, wherein the one or more portions of the one or more signals with cardiac activity are blanked immediately before, during, and/or after individual pulses of the therapeutic electrical signal are applied to the anatomical element.
18. The system of clause 16, wherein the memory stores further data for processing by the processor that, when processed, causes the processor to:
   generate one or more growth curves based at least in part on applying the therapeutic electrical signal to the anatomical element;
   determine a threshold based at least in part on the one or more growth curves, wherein the one or more aggregate cardiac-derived metrics are collected when evoked signals are below the determined threshold; and
   perform signal processing for stimuli that evoke signals larger than the threshold to measure the one or more signals with cardiac activity, wherein the one or more sources of noise are reduced based at least in part on performing the signal processing for stimuli that evoke signals larger than the threshold.
19. A system for identifying and reducing noise in a therapeutic procedure, comprising:
   a pulse generator configured to generate a therapeutic electrical signal;
   one or more leads in communication with the pulse generator and configured to transmit the therapeutic electrical signal to a plurality of electrodes; and
   the plurality of electrodes in communication with the one or more leads, the plurality of electrodes configured to apply the therapeutic electrical signal to an anatomical element of the patient and configured to measure a physiological response,
   wherein the therapeutic electrical signal is applied to the anatomical element according to one or more parameters determined from one or more aggregate cardiac metrics and/or saved processed cardiac data derived from one or more signals with cardiac activity measured via one or more of the plurality of electrodes, the one or more signals with cardiac activity having one or more sources of noise reduced from the one or more signals with cardiac activity.
20. The system of clause 19, wherein the one or more sources of noise are caused by the patient walking or moving, a stimulation artifact, environmental noise, evoked compound action potential activity, evoked compound muscle action potential activity, or a combination thereof.

## Claims

1. A system for identifying and reducing noise in a therapeutic procedure, comprising:
a pulse generator configured to generate a therapeutic electrical signal;
one or more leads in communication with the pulse generator and configured to transmit the therapeutic electrical signal to a plurality of electrodes;
the plurality of electrodes in communication with the one or more leads, the plurality of electrodes configured to apply the therapeutic electrical signal to an anatomical element of a patient and configured to measure a physiological response;
a processor; and
a memory storing data for processing by the processor, the data, when processed, causes the processor to:
measure, via one or more of the plurality of electrodes, one or more signals with cardiac activity of the patient;
identify one or more sources of noise that are distorting the one or more signals with cardiac activity;
reduce the one or more sources of noise from the one or more signals with cardiac activity;
determine one or more aggregate cardiac-derived metrics and/or save processed cardiac data based at least in part on the one or more signals with cardiac activity with the one or more sources of noise reduced; and
determine one or more parameters for applying the therapeutic electrical signal to the anatomical element based at least in part on the one or more aggregate cardiac-derived metrics and/or saved processed cardiac data.

2. The system of claim 1, wherein the memory stores further data for processing by the processor that, when processed, causes the processor to:
process one or more portions of the one or more signals with cardiac activity, the one or more portions corresponding to time points when the one or more sources of noise are expected to occur after the therapeutic electrical signal is applied to the anatomical element.

3. The system of claim 2, wherein the one or more portions of the one or more signals with cardiac activity are blanked immediately before, during, and/or after individual pulses of the therapeutic electrical signal are applied to the anatomical element.

4. The system of claim 2, wherein the one or more portions of the one or more signals with cardiac activity are processed based at least in part on a template subtraction method, spectral filtering, wavelet filtering, or a combination thereof.

5. The system of any of the preceding claims, wherein the memory stores further data for processing by the processor that, when processed, causes the processor to:
generate one or more growth curves based at least in part on applying the therapeutic electrical signal to the anatomical element;
determine a threshold based at least in part on the one or more growth curves, wherein the one or more aggregate cardiac-derived metrics are collected when evoked signals are below the determined threshold; and
perform signal processing for stimuli that evoke signals larger than the threshold to measure the one or more signals with cardiac activity, wherein the one or more sources of noise are reduced based at least in part on performing the signal processing for stimuli that evoke signals larger than the threshold.

6. The system of claim 5, wherein the stimuli that evoke signals larger than the threshold comprise stimulation amplitudes for added processing.

7. The system of any of the preceding claims, wherein the data stored in the memory that, when processed causes the processor to reduce the one or more sources of noise from the one or more signals with cardiac activity further causes the system to:
cycle one or more parameters used for applying the therapeutic electrical signal to the anatomical element and/or one or more parameters for measuring the one or more signals with cardiac activity between 'on' phases and 'off' phases, wherein the one or more signals with cardiac activity are measured during the 'off' phases.

8. The system of claim 7, wherein the 'on' phases comprise applying the therapeutic electrical signal to the anatomical element using a combination or high frequency and/or low frequency stimulations and/or high and/or low amplitudes, and the 'off' phases comprise applying the therapeutic electrical signal to the anatomical element using low frequency stimulations, lower ratios of cycling, low amplitudes, or a combination thereof.

9. The system of any of the preceding claims, wherein the data stored in the memory that, when processed causes the processor to reduce the one or more sources of noise from the one or more signals with cardiac activity further causes the system to:
filter the one or more signals with cardiac activity to reduce the one or more sources of noise.

10. The system of any of the preceding claims, wherein the data stored in the memory that, when processed causes the processor to reduce the one or more sources of noise from the one or more signals with cardiac activity further causes the system to:
identify time points when the one or more signals with cardiac activity have been corrupted by the one or more sources of noise; and
remove portions of the one or more signals with cardiac activity corresponding to the identified time points to reduce the one or more sources of noise from the one or more signals with cardiac activity.

11. The system of claim 10, wherein the time points are identified based at least in part on time intervals when a given R-R interval is non-physiological and/or differs from R-R intervals detected for preceding heartbeats by a defined threshold, when a presence of increased spectral power of greater than a frequency threshold is identified, when changes in a magnitude threshold for the one or more signals with cardiac activity are identified, when movement is detected for the patient, or a combination thereof.

12. The system of any of the preceding claims, wherein the one or more signals with cardiac activity of the patient are measured using a continuously running noise filter, wherein the noise filter is adjusted when the one or more sources of noise meet or exceed a predetermined threshold, wherein the adjusted noise filter uses more power than the non-adjusted noise filter.

13. The system of any of the preceding claims, wherein the memory stores further data for processing by the processor that, when processed, causes the processor to:
output, via a user interface, the one or more aggregate cardiac-derived metrics and/or the saved processed cardiac data, wherein the one or more parameters for applying the therapeutic electrical signal to the anatomical element are determined based at least in part on outputting the one or more aggregate cardiac-derived metrics and/or the saved processed cardiac data.

14. A system for identifying and reducing noise in a therapeutic procedure, comprising:
a processor; and
a memory storing data for processing by the processor, the data, when processed, causes the processor to:
measure, via one or more of a plurality of electrodes, one or more signals with cardiac activity of a patient;
identify one or more sources of noise that are distorting the one or more signals with cardiac activity;
reduce the one or more sources of noise from the one or more signals with cardiac activity;
determine one or more aggregate cardiac-derived metrics and/or save processed cardiac data based at least in part on the one or more signals with cardiac activity with the one or more sources of noise reduced; and
determine one or more parameters for applying a therapeutic electrical signal to an anatomical element based at least in part on the one or more aggregate cardiac-derived metrics and/or saved processed cardiac data.

15. A system for identifying and reducing noise in a therapeutic procedure, comprising:
a pulse generator configured to generate a therapeutic electrical signal;
one or more leads in communication with the pulse generator and configured to transmit the therapeutic electrical signal to a plurality of electrodes; and
the plurality of electrodes in communication with the one or more leads, the plurality of electrodes configured to apply the therapeutic electrical signal to an anatomical element of the patient and configured to measure a physiological response,
wherein the therapeutic electrical signal is applied to the anatomical element according to one or more parameters determined from one or more aggregate cardiac metrics and/or saved processed cardiac data derived from one or more signals with cardiac activity measured via one or more of the plurality of electrodes, the one or more signals with cardiac activity having one or more sources of noise reduced from the one or more signals with cardiac activity.
